# EUROPEAN PATENT APPLICATION

(11) **EP 4 425 500 A2**
(43) Date of publication of application: **04.09.2024**
(21) Application number: 24185655.8
(22) Date of filing: 25.03.2016
(51) Int. Cl.: G16B 30/10

(54) **ALIGNMENT AND VARIANT SEQUENCING ANALYSIS PIPELINE**

(30) Priority: 26.03.2015 US 201562138620 P; 11.11.2015 US 201562253908 P
(62) Divisional of application: 16769805.9
(71) Applicant: Quest Diagnostics Investments Incorporated, Wilmington, DE 19899 (US)
(72) Inventor: Elzinga, Christopher, San Juan Capistrano, California, 92675 (US)
(74) Representative: Hoffmann Eitle

(57) **Abstract**

Provided are systems and methods for analyzing genetic sequence data from next generation sequence (NGS) platforms. Also provided are methods for the preparation of samples for nucleic acid sequence analysis by NGS. Variant calling is performed with a modified GATK variant caller. Mapping the reads to a genomic reference is performed with a Burrows Wheeler Aligner (BWA) and does not comprise soft clipping. The genomic reference sequence is GRCh37.1 human genome reference. The sequencing method comprises emulsion PCR (emPCR), rolling circle amplification, or solid-phase amplification. In some embodiments, the solid-phase amplification is clonal bridge amplification.

## Description

### CROSS-REFERENCE TO RELATED APPLICATIONS

This application claims priority to and the benefit of U.S. Provisional Application Nos. 62/138620, filed March 26, 2015, and 62/253908, filed November 11, 2015, the contents of which are each incorporated herein by reference in their entirety.

### BACKGROUND

Every year, more than 200,000 new cases of breast cancer are diagnosed in the United States. Of these, approximately 2% to 5% are associated with loss-of-function variants in the BRCA1 or BRCA2 genes. With the exception of Ashkenazi-Jewish women, who have a 2% to 5% carrier frequency for 3 founder mutations in BRCA1 and BRCA2, the estimated carrier frequency in the general population is 1:300 for BRCA1 and 1:800 for BRCA2. Patients with deleterious mutations in either the BRCA1 or BRCA2 gene have a 50% to 80% lifetime risk of developing breast cancer and a 20% to 40% lifetime risk of developing ovarian cancer. Triple-negative breast cancers-those that do not express estrogen receptor, progesterone receptor, or Her2/neu and are characterized as being more aggressive-account for 15% to 20% of all breast cancers, they are associated with BRCA mutations at frequencies between 4% and 42%, depending on the characteristics of the study population (e.g., proportion of women who are Ashkenazi Jewish).

The National Comprehensive Cancer Network (NCCN) has developed guidelines for assisting healthcare providers in identifying patients and family members at high risk of breast and ovarian cancer and who may benefit from cancer genetic risk assessment. Genetic risk assessment can include genetic testing but is a dynamic counseling process. Determining whether a woman with breast cancer is BRCA1/2 positive can assist in appropriate counseling regarding increased surveillance and the risks and benefits of undergoing contralateral mastectomy and/or salpingo-oophorectomy, both of which have been shown to be protective against breast cancer. Identifying a deleterious BRCA1/2 variant in a patient can also be helpful to family members, who may need access to genetic counseling and testing to assess their cancer risk and identify appropriate management. The American Society of Breast Surgeons recommends BRCA1/2 testing for individuals from high-risk populations, including those with early onset breast cancer (diagnosed before age 50); two primary breast cancers, either bilateral or ipsilateral; family history of early onset breast cancer; male breast cancer; personal or family history of ovarian cancer (particularly nonmucinous types); Ashkenazi (Eastern European) Jewish heritage in the setting of a newly diagnosed breast cancer or family history of breast cancer; previously identified BRCA1 or BRCA2 mutation in the family; triple-negative breast cancer at ≤60 years of age; or pancreatic cancer associated with a family history of hereditary breast and ovarian related cancer.

Comprehensive BRCA testing typically consists of sequencing all the coding exons and the splice junction regions of BRCA1 and BRCA2 and analysis of large gene rearrangements. PCR-based sequencing methods, including Sanger sequencing and next-generation sequencing (NGS) systems that use PCR amplification, may yield false-negative results due to allele drop-out when polymorphisms are present in amplification or sequencing primer sequences.

Accordingly, there is a need for improved methods for sequencing samples and methods for accurate and efficient analysis of NGS sequencing data.

### SUMMARY

Provided herein in certain embodiments are methods of processing sequencing data generated by high throughput sequencing methods, including next generation sequencing platforms. Exemplary sequencing platforms include, but are not limited to, Illumina MiSeq System and the Life Technologies Ion Torrent Personal Genome Machine.

Provided herein, in certain embodiments, are methods for determining the presence of a variant in one or more genes in a subject comprising: (a) providing raw sequencing data generated from a nucleic acid sequencing reaction on a nucleic acid sample from the subject using a nucleic acid sequencer; (b) removing low quality reads from the raw sequencing data that fail a quality filter, (c) trimming adapter and/or molecular identification (MID) sequences from the filtered raw sequencing data; (d) mapping the filtered raw sequencing data to a genomic reference sequence to generate mapped reads; (e) sorting and indexing the mapped reads; (f) adding read groups to a data file to generate a processed sequence file; (g) creating realigner targets; (h) performing local realignment of the processed sequence file to generate a re-aligned sequence file; (i) removing of duplicate reads from the re-aligned sequence file; (j) analyzing coding regions of interest; and (k) generating a report that identifies whether the variant is present based on the analysis in step (j), wherein steps (g) and (j) are performed using a modified genomic alignment utility limited to nucleic acid regions containing the one or more genes of interest. In some embodiments, the method comprises performing the nucleic acid sequencing reaction on the nucleic acid sample from the subject using a nucleic acid sequencer to generate the raw sequencing data of step (a). In some embodiments, analyzing coding regions of interest comprises calling variants at every position in the regions of interest. In some embodiments, the regions of interest are padded by an additional 150 bases. In some embodiments, variant calling is performed with a modified GATK variant caller. In some embodiments, mapping the reads to a genomic reference sequence is performed with a Burrows Wheeler Aligner (BWA). In some embodiments, mapping the reads to a genomic reference sequence does not comprise soft clipping. In some embodiments, the genomic reference sequence is GRCh37.1 human genome reference. In some embodiments, the sequencing method comprises emulsion PCR (emPCR), rolling circle amplification, or solid-phase amplification. In some embodiments, the solid-phase amplification is clonal bridge amplification.

In some embodiments, the nucleic acid for sequence analysis is extracted from a biological sample from a subject. In some embodiments, the biological sample is a fluid or tissue sample. In some embodiments, the biological sample is a blood sample. In some embodiments, the nucleic acid is genomic DNA. In some embodiments, the nucleic acid is cDNA reversed transcribed from mRNA.

In some embodiments, wherein the nucleic acid samples is prepared prior to sequencing by performing one or more of the following methods: (a) shearing the nucleic acid; (b) concentrating the nucleic acid sample; (c) size selecting the nucleic acid molecule in a sheared nucleic acid sample; (d) repairing ends of the nucleic acid molecules in the sample using a DNA polymerase; (e) attaching one or more adapter sequences; (f) amplifying nucleic acids to increase the proportion of nucleic acids having an attached adapter sequence; (g) enriching the nucleic acid sample for one or more genes of interest; and/or (h) quantification of the nucleic acid sample primer immediately prior to sequencing. In some embodiments, the one or more adapter sequences comprises nucleic acid sequences for priming the sequencing reaction and/or a nucleic acid amplification reaction. In some embodiments, the one or more adapter sequences comprises a molecular identification (MID) tag. In some embodiments, enriching the nucleic acid sample for one or more genes of interest comprises exon capture using one or more biotinylated RNA baits. In some embodiments, the biotinylated RNA baits are specific for exonic regions, splice junction sites, or intronic region or one or more genes of interest. In some embodiments, the one or more biotinylated RNA baits are specific for a BRCA1 gene and/or a BRCA2 gene.

In some embodiments, the nucleic acid for sequence analysis is obtained from a subject that is a mammal. In some embodiments, the subject is a human patient. In some embodiments, the subject is a human suspected of having cancer or suspected of being at risk of developing a cancer. In some embodiments, the cancer is a breast or ovarian cancer.

In some embodiments, one or more variants in a gene associated with a cancer are determined by the methods provided herein. In some embodiments, one or more variants in the BRCA1 gene or BRCA2 gene are determined. In some embodiments, one or more variants is selected from the variants listed in Table 1.

In some embodiments, the methods provided further comprise confirming the presence of the one or more variants by sequencing.

Also provided herein, in certain embodiments, are systems comprising one or more electronic processors configured to: (a) remove low quality reads from the raw sequencing data that fail a quality filter; (b) trim adapter and/or molecular identification (MID) sequences from the filtered raw sequencing data; (c) map the filtered raw sequencing data to a genomic reference sequence to generate mapped reads; (d) sort and index the mapped reads; (e) add read groups to a data file to generate a processed sequence file; (f) create realigner targets; (g) perform local realignment of the processed sequence file to generate a re-aligned sequence file; (h) remove of duplicate reads from the re-aligned sequence file; and (i) analyze coding regions of interest. In some embodiments, analyzing coding regions of interest comprises calling variants at every position in the regions of interest. In some embodiments, the regions of interest are padded by an additional 150 bases. In some embodiments, variant calling is performed with a modified GATK variant caller. In some embodiments, mapping the reads to a genomic reference sequence is performed with a Burrows Wheeler Aligner (BWA). In some embodiments, mapping the reads to a genomic reference sequence does not comprise soft clipping. In some embodiments, the genomic reference sequence is GRCh37.1 human genome reference.

Also provided herein, in certain embodiments, are non-transitory computer-readable media having instructions stored thereon, the instructions comprising: (a) instructions to remove low quality reads that fail a quality filter; (b) instructions to trim adapter and MID sequences from the filtered raw sequencing data; (c) instructions to map the filtered raw sequencing data to a genomic reference sequence to generate mapped reads; (d) instructions to sort and index the mapped reads; (e) instructions to add read groups to a data file to generate a processed sequence file; (f) instructions to create realigner targets; (g) instructions to perform local realignment of the processed sequence file to generate a re-aligned sequence file; (h) instructions to remove duplicate reads from the re-aligned sequence file; and (i) instructions to analyze coding regions of interest. In some embodiments, analyzing coding regions of interest comprises calling variants at every position in the regions of interest. In some embodiments, the regions of interest are padded by an additional 150 bases. In some embodiments, variant calling is performed with a modified GATK variant caller. In some embodiments, mapping the reads to a genomic reference sequence is performed with a Burrows Wheeler Aligner (BWA). In some embodiments, mapping the reads to a genomic reference sequence does not comprise soft clipping. In some embodiments, the genomic reference sequence is GRCh37.1 human genome reference.

The foregoing summary is illustrative only and is not intended to be in any way limiting. In addition to the illustrative aspects, implementations, and features described above, further aspects, implementations, and features will become apparent by reference to the following drawings and the detailed description.

### BRIEF DESCRIPTION OF THE DRAWINGS

The foregoing and other features of the present disclosure will become more fully apparent from the following description and appended claims, taken in conjunction with the accompanying drawings. Understanding that these drawings depict only several implementations in accordance with the disclosure and are, therefore, not to be considered limiting of its scope, the disclosure will be described with additional specificity and detail through use of the accompanying drawings.

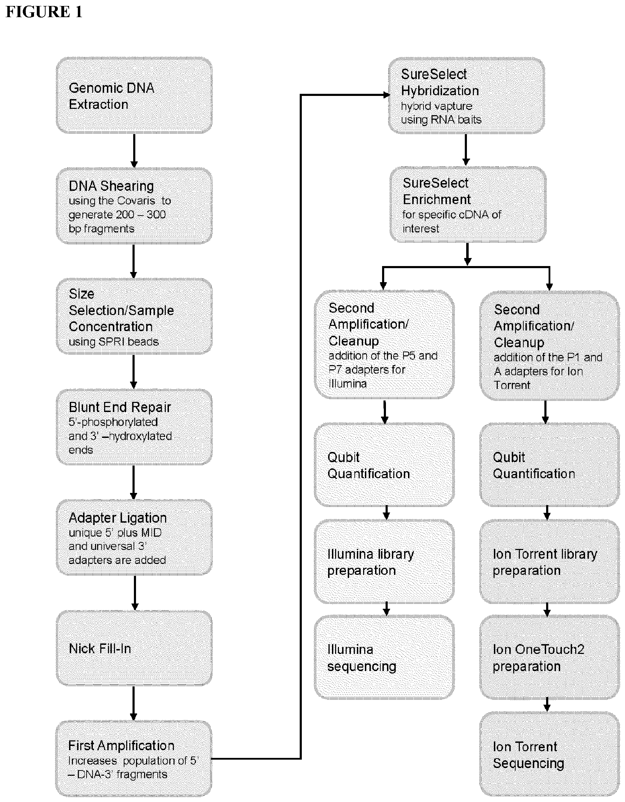

FIG. 1 illustrates an exemplary general overview of the NGS assay for detection of *BRCA1* and *BRCA2* variants in accordance with various illustrative implementations using the MiSeq and the Personal Gene Machine (PGM) platforms. For the MiSeq platform, variant calling was performed initially with the vendor-supplied MiSeq Reporter software and then with the Quest Sequencing Analysis Pipeline (QSAP). For the PGM platform, the vendor-supplied Torrent Suite variant-calling software was used.

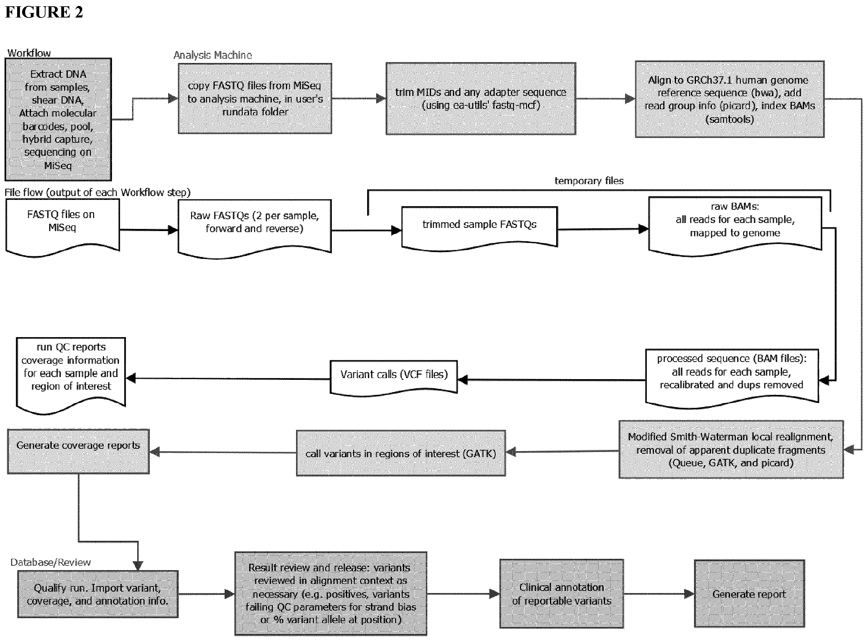

FIG. 2 is a flow diagram of the QSAP bioinformatics sequence analysis methods in accordance with various illustrative implementations.

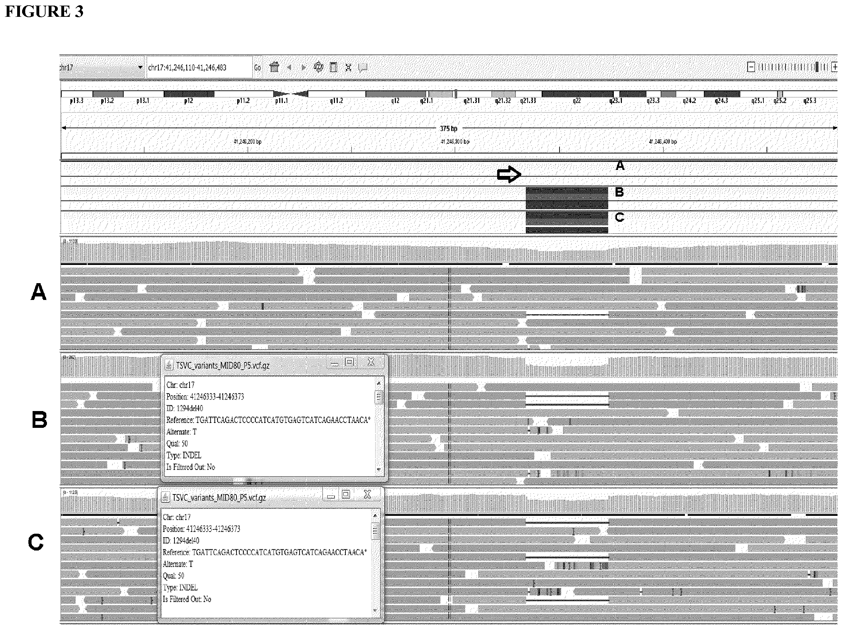

FIG. 3 shows an exemplary alignment of a 40-bp deletion in *BRCA1* (deletion c.1175_1214del40) in a validation sample. The Integrative Genomics Viewer (IGV) graphic report shows detection of the mutation with the PGM platform with Torrent Suite variant calling (panel A) but not the MiSeq platform with MiSeq Reporter (panel B). Use of QSAP with the MiSeq platform allowed detection of the deletion (panel C).

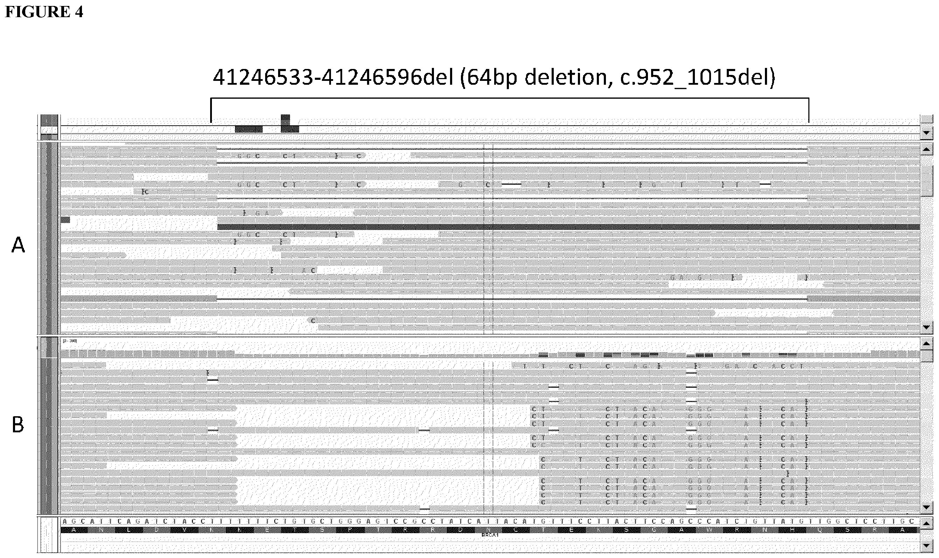

FIG. 4 shows an exemplary alignment of a 64 bp-deletion (41246533-41246596del; c.952_1015del) in a validation sample. The Integrative Genomics Viewer (IGV) graphic reports show detection of the deletion using MiSeq platform with QSAP (panel A) but not the PGM platform with Torrent Suite variant calling software (panel B).

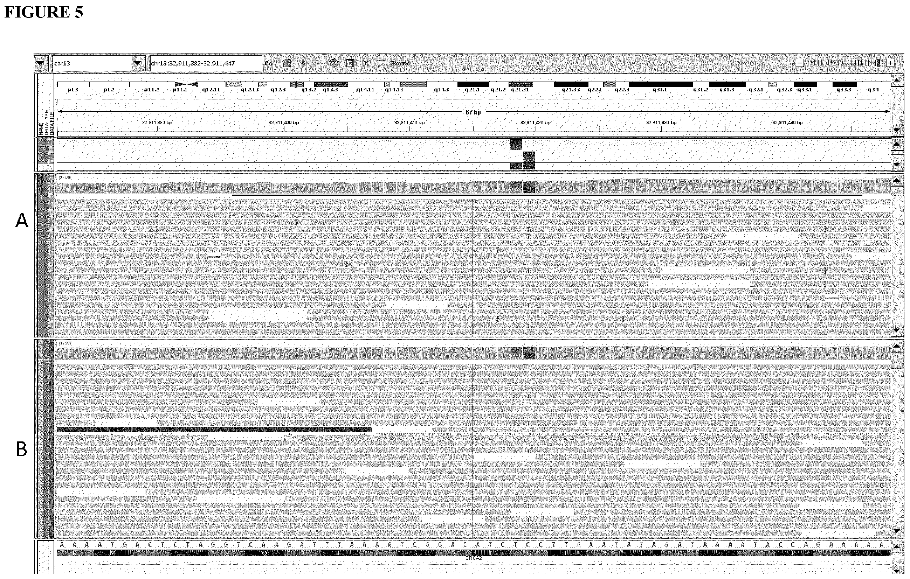

FIG. 5 shows an exemplary determination of *cis* vs. *trans* orientation using next-generation sequencing (NGS). The Integrative Genomics Viewer (IGV) graphic report is from a patient with 2 adjacent variants on a single DNA molecule visualized with NGS on the MiSeq/QSAP platform. The *cis* orientation is clearly visible, as each strand contains either both or neither of the mutations.

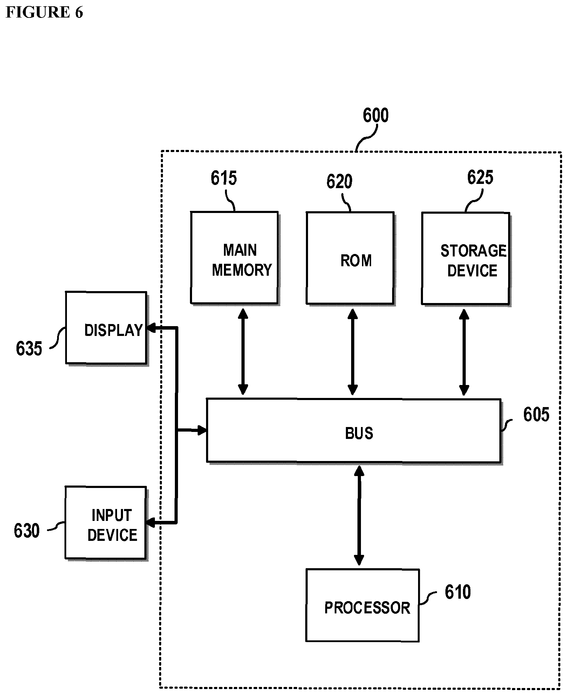

FIG. 6 is a diagram of a computing system that may be used in conjunction with the methods provided herein.

Reference is made to the accompanying drawings throughout the following detailed description. In the drawings, similar symbols typically identify similar components, unless context dictates otherwise. The illustrative implementations described in the detailed description, drawings, and claims are not meant to be limiting. Other implementations may be utilized, and other changes may be made, without departing from the spirit or scope of the subject matter presented here. It will be readily understood that the aspects of the present disclosure, as generally described herein, and illustrated in the figures, can be arranged, substituted, combined, and designed in a wide variety of different configurations, all of which are explicitly contemplated and made part of this disclosure.

### DETAILED DESCRIPTION

### Certain Terminology

Certain terms employed in this description have the following defined meanings. Terms that are not defined have their art-recognized meanings. That is, unless otherwise defined, all technical and scientific terms used herein have the same meaning as commonly understood by one of ordinary skill in the art to which this invention belongs.

As used herein, unless indicated otherwise, when referring to a numerical value, the term "about" means plus or minus 10% of the enumerated value.

As used herein, the terms "isolated," "purified" or "substantially purified" refer to molecules, such as nucleic acid, that are removed from their natural environment, isolated or separated, and are at least 60% free, preferably 75% free, and most preferably 90% free from other components with which they are naturally associated. An isolated molecule is therefore a substantially purified molecule.

A "fragment" in the context of a gene fragment or a chromosome fragment refers to a sequence of nucleotide residues which are at least about 10 nucleotides, at least about 20 nucleotides, at least about 25 nucleotides, at least about 30 nucleotides, at least about 40 nucleotides, at least about 50 nucleotides, at least about 100 nucleotides, at least about 250 nucleotides, at least about 500 nucleotides, at least about 1,000 nucleotides, at least about 2,000 nucleotides.

The terms "identity" and "identical" refer to a degree of identity between sequences. There may be partial identity or complete identity. A partially identical sequence is one that is less than 100% identical to another sequence. Partially identical sequences may have an overall identity of at least 70% or at least 75%, at least 80% or at least 85%, or at least 90% or at least 95%.

The terms "amplification" or "amplify" as used herein includes methods for copying a target nucleic acid, thereby increasing the number of copies of a selected nucleic acid sequence. Amplification may be exponential or linear. A target nucleic acid may be either DNA or RNA. The sequences amplified in this manner form an "amplification product," also known as an "amplicon." While the exemplary methods described hereinafter relate to amplification using the polymerase chain reaction (PCR), numerous other methods are known in the art for amplification of nucleic acids (e.g., isothermal methods, rolling circle methods, etc.). The skilled artisan will understand that these other methods may be used either in place of, or together with, PCR methods. See, e.g., Saiki, "Amplification of Genomic DNA" in PCR Protocols, Innis et al., Eds., Academic Press, San Diego, CA 1990, pp. 13-20; Wharamet al., Nucleic Acids Res., 29(11):E54-E54, 2001; Hafneret al., Biotechniques, 30(4):852-56, 858, 860, 2001, Zhonget al., Biotechniques, 30(4):852-6, 858, 860, 2001.

The term "detectable label" as used herein refers to a molecule or a compound or a group of molecules or a group of compounds associated with a probe and is used to identify the probe hybridized to a genomic nucleic acid or reference nucleic acid.

As used herein, the term "detecting" refers to observing a signal from a detectable label to indicate the presence of a target. More specifically, detecting is used in the context of detecting a specific sequence.

The term "high throughput, massively parallel sequencing" as used herein refers to sequencing methods that can generate multiple sequencing reactions of clonally amplified molecules and of single nucleic acid molecules in parallel. This allows increased throughput and yield of data. These methods are also known in the art as next generation sequencing (NGS) methods. NGS methods include, for example, sequencing-by-synthesis using reversible dye terminators, and sequencing-by-ligation. Non-limiting examples of commonly used NGS platforms include miRNA Bead Array (Illumina, Inc.), Roche 454TM GS FLXTM-Titanium (Roche Diagnostics), ABI SOLiDTM System (Applied Biosystems, Foster City, CA), and HeliScope^{™} Sequencing System (Helicos Biosciences Corp., Cambridge MA).

"Sequencing depth" or "read depth" as used herein refers to the number of times a sequence has been sequenced (the depth of sequencing). As an example, read depth can be determined by aligning multiple sequencing run results and counting the start position of reads in nonoverlapping windows of a certain size (for example, 100 bp). Copy number variation can be determined based on read depth using methods known in the art. For example, using a method described in Yoon et al., Genome Research 2009 September; 19(9): 1586-1592; Xie et al., BMC Bioinformatics 2009 Mar 6; 10:80; or Medvedev et al., Nature Methods 2009 Nov; 6(11 Suppl): S13-20. Use of this type of method and analysis is referred to as a "read depth approach."

A "nucleic acid fragment read" as used herein refers to a single, short contiguous information piece or stretch of sequence data A read may have any suitable length, for example a length of between about 30 nucleotides to about 1000 nucleotides. The length generally depends on the sequencing technology used for obtaining it. In specific embodiments, the reads may also be longer, e.g. 2 to 10 kb or more. The present methods generally envisage any read or read length and is not to be understood as being limited to the presently available read lengths, but also includes further developments in this area, e.g. the development of long reading sequencing approaches etc.

A "nucleic acid sequence data" as used herein may be any sequence information on nucleic acid molecules known to the skilled person. The sequence data can include information on DNA or RNA sequences, modified nucleic acids, single strand or duplex sequences, or alternatively amino acid sequences, which have to converted into nucleic acid sequences. The sequence data may additionally comprise information on the sequencing machine, date of acquisition, read length, direction of sequencing, origin of the sequenced entity, neighboring sequences or reads, presence of repeats or any other suitable parameter known to the person skilled in the art. The sequence data may be presented in any suitable format, archive, coding or document known to the person skilled in the art. The data may, for example, be in the format of FASTQ, Qseq, CSFASTA, BED, WIG, EMBL, Phred, GFF, SAM, SRF, SFF or ABI-ABIF.

The phrase "obtaining sequence data from a plurality of nucleic acid fragment reads" as used herein refer to the process of determining the sequence information of a subject, or a group of subjects by the performance of nucleic acid sequencing reactions.

The term "multiplex PCR" as used herein refers to an assay that provides for simultaneous amplification and detection of two or more target nucleic acids within the same reaction vessel. Each amplification reaction is primed using a distinct primer pair. In some embodiments, at least one primer of each primer pair is labeled with a detectable moiety. In some embodiments, a multiplex reaction may further include specific probes for each target nucleic acid. In some embodiments, the specific probes are detectably labeled with different detectable moieties.

The term "nested polymerase chain reaction" is a modification of polymerase chain reaction which, in the present context, is performed to add sequences to an amplicon. Nested polymerase chain reaction involves two sets of primers, used in two successive runs of polymerase chain reaction, the second set intended to amplify the target from the first run product.

As used herein, the term "oligonucleotide" refers to a short polymer composed of deoxyribonucleotides, ribonucleotides, or any combination thereof. Oligonucleotides are generally between about 10, 11, 12, 13, 14, 15, 20, 25, or 30 to about 150 nucleotides (nt) in length, more preferably about 10, 11, 12, 13, 14, 15, 20, 25, or 30 to about 70 nt.

The term "specific" as used herein in reference to an oligonucleotide primer means that the nucleotide sequence of the primer has at least 12 bases of sequence identity with a portion of the nucleic acid to be amplified when the oligonucleotide and the nucleic acid are aligned. An oligonucleotide primer that is specific for a nucleic acid is one that, under the stringent hybridization or washing conditions, is capable of hybridizing to the target of interest and not substantially hybridizing to nucleic acids which are not of interest. Higher levels of sequence identity are preferred and include at least 75%, at least 80%, at least 85%, at least 90%, at least 95% and more preferably at least 98% sequence identity.

As used herein, the term "subject" or "individual" refers to a mammal, such as a human, but can also be another animal such as a domestic animal (e.g., a dog, cat, or the like), a farm animal (e.g., a cow, a sheep, a pig, a horse, or the like) or a laboratory animal (e.g., a monkey, a rat, a mouse, a rabbit, a guinea pig, or the like).

The terms "complement," "complementary" or "complementarity" as used herein with reference to polynucleotides (i.e., a sequence of nucleotides such as an oligonucleotide or a genomic nucleic acid) related by the base-pairing rules. The complement of a nucleic acid sequence as used herein refers to an oligonucleotide which, when aligned with the nucleic acid sequence such that the 5' end of one sequence is paired with the 3' end of the other, is in "antiparallel association." For example, for the sequence 5'-A-G-T-3' is complementary to the sequence 3'-T-C-A-5'. Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs. Complementarity may be "partial" in which only some of the nucleic acids' bases are matched according to the base pairing rules. Or, there may be "complete," "total," or "full" complementarity between the nucleic acids.

"Detecting" a mutation in a gene or protein may be accomplished by performing an appropriate assay. To detect a mutation in a gene or protein in a biological sample, the biological sample is assayed to determine the presence or absence of the mutated gene or mutated protein. The assay may include extracting nucleic acid (such as, for example, total genomic DNA and/or RNA) from the sample and analyzing the extracted nucleic acid by methods known in the art. An assay may involve isolating protein from the biological sample and analyzing the protein. However, an assay need not involve either extraction of nucleic acid or isolation of protein. That is, some assays may be employed that directly analyze a biological sample without extracting or isolating nucleic acid or protein.

As used herein, the term "subject" refers to a mammal, such as a human, but can also be another animal such as a domestic animal (e.g., a dog, cat, or the like), a farm animal (e.g., a cow, a sheep, a pig, a horse, or the like) or a laboratory animal (e.g., a monkey, a rat, a mouse, a rabbit, a guinea pig, or the like). The term "patient" refers to a "subject" who possesses, or is suspected to possess, a genetic polymorphism of interest.

### Overview

Genomic sequence analysis using the Illumina MiSeq sequencing system using the supplied MiSeq Reporter software for the detection of BRCA1 and BRCA2 variants is unable to detect certain key mutations. Specifically, the MiSeq sequencing system has a lower sensitivity for certain types of variants, such as medium-sized insertions or deletions. For example, as described herein in the working examples, the MiSeq sequencing system was unable to identify two pathological *BRCA1* variants having deletions of greater than 9 base pairs (bp): the 40-bp deletion c.1175_1214del40 and the 10-bp deletion c.3481_3491del10. A sequence data analysis pipeline (QSAP for Quest Sequence Analysis Pipeline) for processing raw sequence data generated from next generation sequence (NGS) platforms was developed for the detection of *BRCA1* and *BRCA2* variants and is described herein. The QSAP workflow as described herein can be used in conjunction with various NGS platforms for alignment and allele assignment. In some embodiments, the QSAP method is able to identify variants that are missed by the MiSeq Reporter software.

With respect to a genomic reference sequence, different fragments in a sheared genomic library tend to start and/or end at different locations than the start and/or end locations of other fragments. The QSAP workflow provided herein involves mitigation of possible effects of amplification bias by removing apparent PCR clones, thereby better recovering the allele balance present in the original sheared genomic sample. Therefore, the bioinformatics analyses are able to differentiate among reads from the same versus different library clones. In addition, the QSAP workflow involves local realignment limited to target regions of interest, which improves sensitivity of detection for insertions and deletions and a quicker, more efficient analysis.

In addition, the use of bait tile library exon capture followed by NGS can avoid potential causes of false-negative testing due to allele drop-out when polymorphisms are present in amplification or sequencing primer sequences Bait tiles are biotinylated RNA molecules approximately 125-bp in length used to capture relevant fragments. Since the bait tiles are about 100 bases longer than typical PCR or sequencing primers and RNA/DNA hybrids are stronger than DNA/DNA hybrids, polymorphisms are less likely to interfere with the exon capture. A second advantage of bait tile capture versus PCR based sequencing methods is the avoidance of false positive results due to clonal bias in PCR or library formation.

### Target Genes and Variants

The systems and methods provided herein can be applied to the detection of a variant in any gene of interest. Exemplary variants include single nucleotide polymorphisms, point mutations, insertions, deletions, and translocations. In some exemplary embodiments, the system and methods provided herein are used for the detection of genomic deletions of greater than 5, 6, 7, 8, 9, 10, 11, 12, 13, 14, 15, 16, 17, 18, 19, 20, 25, 30, 35, 40, 45, 50 or greater base pairs.

In some exemplary embodiments, the gene of interest is a *BRCA1* gene or a *BRCA2* gene. A *BRCA1* or *BRACA2* target segment that is sequenced may represent all or part of a *BRCA1* or *BRACA2* genomic DNA or cDNA. In some embodiments, one or more *BRCA1* or *BRACA2* exons or portions thereof are sequenced. In some embodiments, one or more of the *BRCA1* or *BRACA2* introns or portions thereof are sequenced. In some embodiments, from at least one, two, five, 10 or 20 up to 25 or 27 exons are sequenced. In other embodiments, all or a portion of the *BRCA1* or *BRACA2* promoter region is also evaluated.

In some embodiments the target segments represent the entire coding and noncoding sequences of the *BRCA1* or *BRACA2* gene. In one embodiment, the *BRCA1* or *BRACA2* target segments, when combined, represent the *BRCAI* or *BRACA2* coding region and all introns, plus from about 100, 500, 750, 900 or 1000 up to about 1200 nucleotides of the *BRCA1* or *BRACA2* promoter immediately upstream (in the 5 prime direction) of the first exon plus from about 50, 100, 150 or 200 up to about 200, 250, 300 or 400 nucleotides immediately downstream (in the 3 prime direction) of the *BRCA1* or *BRACA2* gene. In some embodiments, the adjacent, upstream region comprises all or a portion of the *BRCA1* or *BRACA2* promoter sequence. In another embodiment, all exons and a portion of one or more introns of *BRCA1* or *BRACA2* are represented.

In some embodiments the variant for detection is a pathogenic mutation in *BRCA1* gene or a *BRCA2* gene. In some embodiments, the pathogenic mutation is selected from a mutation provided in Table 1. In some embodiments the variant for detection is c.1175_1214del40 deletion or a c.3481_3491del10 deletion in the BRCA1 gene.

**Table 1. BRCA1 and BRCA2 Variants in the 27 Coriell Cell Line Reference Samples**

| Sample | dbSNP HGVS names | |
|---|---|---|
| | NM_007300.3 | NP_009231.2 |
| ***BRCA1*** | | |
| GM13711 | c.3119G>A | p.Ser1040Asn |
| GM13715 | c.5326_5327insC | p.Ser1776delinsSerProfs |
| GM14634 | c.4065_4068delTCAA | p.Asn1355_Gln1356delinsLysfs |
| GM14636 | c.5621_5622insA | p.Tyr1874delinsTerProfs |
| GM14637 | c.4327C>T | p.Arg1443Ter |
| GM14638 | c.213-11T>G | - |
| GM14684 | c.797_798delTT | p.Val266=fs |
| GM14090 | c.66_67delAG | p.Leu22_Glu23delinsLeuValfs |
| GM14092 | c.5201T>C | p.Val1734Ala |
| GM14093 | c.1204delG | p.Glu402Serfs |
| GM14094 | c.1175_1214del40 | p.Leu392_Ser405delinsGlnfs |
| GM14095 | c.5200delG | p.Val1734Terfs |
| GM14096 | c.3481_3491delGAAGATACTAG | p.Glu1161_Ser1164delinsPhefs |
| GM14097 | c.181T>G | p.Cys61Gly |
| GM13714 | c.5382_5383insC | p.Asn1795Glnfs |
| GM13713 | c.3748G>T | p.Glu1250Ter |
| GM13712 | c.2155_2156insA | p.Lys719delinsLysArgfs |
| GM13710 | c.4327C>G | p.Arg1443Gly |
| GM13709 | c.2068delA | p.Lys690=fs |
| GM13708 | c.4752C>G | p.Tyr1584Ter |
| GM13705 | c.3756_3759delGTCT | p.Leu1252 Ser1253delinsLeufs |

| ***BRCA2*** | | |
|---|---|---|
| GM14170 | c.5946delT | p.Ser1982Argfs |
| GM14622 | c.6275_6276delTT | p.Leu2092Profs |
| GM14623 | c 125A>G | p.Tyr42Cys |
| GM14624 | c.5718_5719delCT | p.Asn1906_Ser1907=fs |
| GM14626 | c.9976A>T | p.Lys3326Ter |
| GM14639 | c.6198_6199delTT | p.Val2066_Ser2067delinsValHisfs |

| | | |
|---|---|---|
| All mutations were detected by NGS with the PGM system and the MiSeq system (with QSAP variant calling) software, as well as by Sanger sequencing. | | |

### Samples for Analysis and Sample Preparation

The methods provided herein can be applied to any nucleic acid obtained from a biological sample. The term "biological sample" as used herein refers to a sample containing a nucleic acid of interest. A biological sample may comprise a clinical sample (i.e., obtained directly from a patient) or isolated nucleic acids and may be cellular or acellular fluids and/or tissue (e.g., biopsy) samples. In some embodiments, a sample is obtained from a tissue or bodily fluid collected from a subject. Sample sources include, but are not limited to, sputum (processed or unprocessed), bronchial alveolar lavage (BAL), bronchial wash (BW), whole blood or isolated blood cells of any type (e.g., lymphocytes), bodily fluids, cerebrospinal fluid (CSF), urine, plasma, serum, or tissue (e.g., biopsy material). Methods of obtaining test samples and reference samples are well known to those of skill in the art and include, but are not limited to, aspirations, tissue sections, drawing of blood or other fluids, surgical or needle biopsies, collection of paraffin embedded tissue, collection of body fluids, collection of stool, and the like. In the present context the biological sample preferably is blood, serum or plasma. The term "patient sample" as used herein refers to a sample obtained from a human seeking diagnosis and/or treatment of a disease or determining the likelihood of developing a disease.

Exemplary methods for the preparation of a genomic DNA sample include, but are not limited to DNA isolation, genomic DNA shearing, measuring DNA concentration, DNA end repair, adaptor ligation, amplification, and enrichment methods, e.g. bait capture methods. An exemplary process flow for the preparation of the DNA sample is provided in Figure 1 and further described in the examples provided herein.

In exemplary embodiments, genomic DNA is isolated from a patient sample and randomly sheared into genomic DNA fragments having an average size of about 250 base pairs. In some embodiments, the isolated genomic DNA can be further purified and concentrated. For example, the isolated genomic DNA can be purified on a solid support such as solid-phase reversible immobilization (SPRI) beads.

In exemplary embodiments, nucleic acid adaptors are added to the 5' and 3' ends of genomic DNA fragments. In some embodiments, at least one adapter contains a unique index sequence (also referred to as an index tag, a "barcode" or a multiplex identifier (MID)) that is used to identify individual DNA samples. Indexed nucleic acids from more than one sample source can be quantified individually and then pooled prior to sequencing. As such, the use of index sequences permits multiple samples (i.e., samples from more than one sample source) to be pooled per sequencing run and the sample source subsequently ascertained based on the index sequence.

In addition, the adaptors may comprise universal sequences useful for priming for amplification and/or sequencing reactions. In some embodiments, the genomic DNA fragments are amplified prior to sequencing. In some embodiments, the adapter sequences are P5 and/or P7 adapter sequences that are recommended for Illumina sequencers (MiSeq and HiSeq). See, e.g., Williams-Carrier et al., Plant J., 63(1):167-77 (2010). In some embodiments, the adapter sequences are P1 or A adapter sequences that are recommended for Life Technologies sequencers. Other adapter sequences are known in the art. Some manufacturers recommend specific adapter sequences for use with the particular sequencing technology and machinery that they offer.

The adaptors can be attached by ligation reaction or via amplification using adapter-ligated and/or indexed primers. When adapter-ligated and/or indexed primers are employed to amplify a target segment, the adapter sequence and/or index sequence gets incorporated into the amplicon (along with the target-specific primer sequence) during amplification.

In exemplary embodiments, particular sequence targets are enriched. Many methods are available for sequence selection. In one example, suitable nucleic acid probes are immobilized on a solid support as a bait to capture polynucleotide fragments having complementary sequences For example, selected target regions of the genome can be enriched by generating a pool of genomic DNA fragments by nucleic acid shearing and sequence-specific capture with nucleic acid probes (i.e. baits). The baits can be complementary to one or more exons, introns and/or splice junction sites located within the region or regions of interest. In some embodiments, the baits are RNA baits. In some embodiments, the baits are tagged (e.g. biotinylated) to facilitate purification of the genomic fragments (e.g. purification with streptavidin- coated beats to adsorb the biotinylated baits). In some embodiments, the baits are biotinylated RNA baits. In some embodiments, the baits are biotinylated RNA baits complimentary to one or more exons, introns and/or splice junction sites located within the *BRCA1* and/or *BRCA2* genes.

Prior to sequencing, additional sequences required for NGS sequencing, for example the Illumina MiSeq^{™} (Illumina, San Diego, CA) or Ion Torrent^{™} Personal Gene Machine (PGM) (Life Technologies, Grand Island, NY) sequencing platforms, can be added to the 5' and 3 ` adaptors by amplification using primers specific for the adaptor sequences.

Control samples from cell lines or nucleic acid samples with known deleterious variants in the gene or genes of interest can be employed for comparison to a test sample containing a nucleic acid of unknown sequence.

### NGS Sequencing Platforms

Generation of sequence data is typically performed using a high throughput DNA sequencing system, such as a next generation sequencing (NGS) system, which employs massively parallel sequencing of DNA templates. Exemplary NGS sequencing platforms for the generation of nucleic acid sequence data include, but are not limited to, Illumina's sequencing by synthesis technology (e.g., Illumina MiSeq or HiSeq System), Life Technologies' Ion Torrent semiconductor sequencing technology (e.g., Ion Torrent PGM or Proton system), the Roche (454 Life Sciences) GS series and Qiagen (Intelligent BioSystems) Gene Reader sequencing platforms.

Generally, two methods are used in preparing templates for NGS reactions: amplified templates originating from single DNA molecules, and single DNA molecule templates. For imaging systems which cannot detect single fluorescence events, amplification of DNA templates is required. The three most common amplification methods are emulsion PCR (emPCR), rolling circle and solid-phase amplification.

In clonal bridge amplification methods, which is used in Illumina HiSeq and MiSeq Systems, forward and reverse primers are covalently attached at high-density to the slide in a flow cell. The ratio of the primers to the template on the support defines the surface density of the amplified clusters. The flow cell is exposed to reagents for polymerase-based extension, and priming occurs as the free/distal end of a ligated fragment "bridges" to a complementary oligo on the surface. Repeated denaturation and extension results in localized amplification of DNA fragments in millions of separate locations across the flow cell surface. Solid-phase amplification produces about 12-15 million (MiSeq) spatially separated template clusters (or about 100-200 million for HiSeq), providing free ends to which a universal sequencing primer is then hybridized to initiate the sequencing reaction. Sequencing is then generally carried out using a reversible dye terminator method, which uses reversible terminator-bound dNTPs in a cyclic method that comprises nucleotide incorporation, fluorescence imaging and cleavage. A fluorescently-labeled terminator is imaged as each dNTP is added and then cleaved to allow incorporation of the next base. These nucleotides are chemically blocked such that each incorporation is a unique event. An imaging step follows each base incorporation step, then the blocked group is chemically removed to prepare each strand for the next incorporation by DNA polymerase. This series of steps continues for a specific number of cycles, as determined by user-defined instrument settings.

In emulsion PCR methods, a DNA library is first generated through random fragmentation of genomic DNA. Single-stranded DNA fragments (templates) are attached to the surface of beads with adaptors or linkers, and one bead is attached to a single DNA fragment from the DNA library. The surface of the beads contains oligonucleotide probes with sequences that are complementary to the adaptors binding the DNA fragments. The beads are then compartmentalized into water-oil emulsion droplets. In the aqueous water-oil emulsion, each of the droplets capturing one bead is a PCR microreactor that produces amplified copies of the single DNA template. These beads are then used to generate the sequence. The beads used in the amplification solution are covered with covalently bound oligonucleotides that are antisense to the P1 sequence of the library. Micro-chambers are created by the Ion Torrent One Touch Instrument 2 (OT2, Life Technologies, Grand Island, NY), which carries out clonal amplification. The strand of DNA that results from extension of the anti-sense P1 oligonucleotide is then hybridized at its 3' end with a sequencing primer that binds at the anti-sense A oligonucleotide. DNA polymerase is added to the beads and then the beads are deposited into tiny pores on the surface of a computer chip-like surface. Each of the four dNTPs is then sequentially flowed in excess over the surface of the chip. The DNA polymerase extends the growing strand when the required nucleotide is made available. Whenever a nucleotide is added, a hydrogen molecule is released resulting in a pH change in the pore containing the sequencing bead. The magnitude of the pH change is approximately equal to the number of nucleotides incorporated and is detected and measured along with which of the four nucleotides that flowed through.

### QSAP Methods of Processing data

Figure 2 illustrates a flow diagram in accordance with various illustrative implementations for the sequence data analysis (i.e., QSAP, Quest Sequence Analysis Pipeline). The process shown in Figure 2 can be implemented on a computing device. In one implementation, the process is encoded on a computer-readable medium that contains instructions that, when executed by a computing device, cause the computing device to perform operations of process.

The raw nucleic acid sequencing data from a nucleic acid sequencer, for example from an NGS sequencing platform, which generates a plurality of nucleic acid sequence data from a plurality of nucleic acid fragment reads. Preferably, the data or data sets are present in one data format, more preferably in a unified data format, e.g. in the FASTQ format, along with their base quality either in Phred/Phrap or modified format. It is preferred that the data format at least covers the sequence read and its associated base quality. In some embodiments, the plurality of raw sequence data may be converted into a unified format.

This raw nucleic acid sequencing data is run through the QSAP method provided herein, which comprises an improved process for sequence alignment and variant calling, leading to improved detection of sequence variants. The QSAP method uses a high performance computing infrastructure, which a combination of open source and modified sequencing tools. The method includes the Burrows Wheeler Aligner (BWA) for alignment mapping to a reference sequence, for example, the hg19/GRCh37.1 reference genome, Queue with the Genome Analysis Tool Kit (GATK) for deduplication, a modified Smith-Waterman local realignment, and forced variant calling (e.g., modified GATK for variant calling). In one embodiment, soft clipping during alignment mapping is turned off. For example, the bwa tool can be run with no soft clipping. A soft-clipped sequence is an unmatched fragment in a partially mapped read. In some embodiments, removing soft clipping eliminates errors due to incorrect mapping of reads that are soft clipped. Local realignment increases the sensitivity of mutation detection, including detection of large inserts and deletions. The pipeline is designed to maximize the accuracy of variant calls, reduce time of analysis and permit ready access to sample Binary alignment/Map format (BAM) and Variant Call Format (VCF) files. Exemplary embodiments for each step of QSAP method is provided herein below. The "reference sequence" as used herein may be any suitable preexisting sequence covering the stretch, which is identical or similar to the newly obtained sequence data or nucleic acid fragment reads.

In exemplary methods, sample nucleotide sequence files, for example FASTQ files, are first copied from the nucleic acid sequencer, (e.g., MiSeq instrument) into a user rundata folder. The sequences are de-multiplexed using index reads on-instrument. The raw FASTQ files comprise at least 2 reads per sample, e.g., forward and reverse reads.

The sample FASTQ files are then filtered to remove reads flagged as failing a vendor quality filter In some embodiments, the filter is a base quality, coverage, complexity of the surrounding region or length of mismatch filter. The filtering procedure can be performed using a sequence sorting utility, such as, for example, SAMtools (Sequence Alignment/Map tools).

The adapter and Molecular Identifier (MID) tag sequences (i.e. identifier sequences ligated to the sample's sequence during the nucleic acid sample preparation process) are then trimmed from the sequence reads. In one implementation, this process is done using a FASTQ processing utility, such as fastq-mcf, which scans a sequence file for the adapters and tag sequences, and, based on a log-scaled threshold, determines a set of clipping parameters and performs clipping. This step produces a file containing the trimmed sample FASTQ reads. The sorted samples have a unique combined adaptor and MID sequence. Thus, trimming is performed according the identity of the adaptor and sample specific-MID sequences to ensure the both the adapter and MID sequences are removed during the trimming process.

The trimmed reads are then mapped to a reference sequence (e.g., a GRCh37.1 human genome reference) suitable reference alignment algorithm, sorted and indexed. This part of the process can be performed using a Burrows-Wheeler Aligner (BWA) (e.g. version 0.7.5a-r405) (e.g., using the command: bwa mem -M -t 2) and SAMtools. In some embodiments, soft clipping during alignment mapping is turned off. Alternative basic alignment tools (e.g., bowtie) can also be employed for the initial alignment. Read groups can then be added to the BAM file using a utility, such as Picard, which comprises Java-based command-line utilities for manipulating SAM and BAM format files. The output of this step is a raw BAM file that contains all reads for each sample mapped to the genome. While SAMtools represents a standard utility for the sorting and indexing of BAM files, other utilities that can process BAM files can be employed. Details and ways of implementing these alignment algorithms are known to the person skilled in the art, or can be derived from suitable literature sources, e.g. from Bao et al., Journal of Human Genetics, 28 Apr. 2011, p. 1-9, which is incorporated herein by reference in its entirety. The present invention further envisages the use of optimized or further developed versions of these algorithms, or of reference alignment algorithms following a different scheme or algorithmic logic including not yet available algorithms, as long as the principle purpose of an alignment to a reference sequence as described herein is fulfilled.

The next step of the process involves pipelined realigner target creation, local realign, and flagging of duplicate reads. In some implementations, Queue can be used for these steps. In one exemplary implementation, the Queue pipeline is a modified Queue pipeline based on the DataProcessingPipeline scala script included in the Queue v.2.3-9 distribution. In some exemplary embodiments, modifications include one or more for the following: the memory and thread usage parameters are customized to fit the computing platform, the modified Smith-Waterman local realign option is used, the base quality score recalibration step (BQSR) is removed, and/or the Queue analysis and BAM output is limited to the regions of interest (e.g., coding exons +/- about 50 bases) optionally padded by additional bases, e.g. about 150 bases. In some embodiments, removal of apparent duplicate fragments (i.e., deduplication) is performed using picard's MarkDuplicates application, which can be pipelined using Queue. The output of these steps is a processed sequence BAM file that contains all reads for each sample, realigned to the region of interest and with duplicates removed.

In some embodiments, the base quality score recalibration (BQSR) is removed to improve compute time constraints. Such modification is useful in instances where a relatively small region of interest is sequenced, e.g., a few selected genes of interest. When focusing on smaller regions of interest, BQSR does not function as well because it relies on having a broad enough sampling of the genome that common variants (e.g. if the reference sequence contains a rare allele, most samples will have a variant at that position) do not interfere with the recalibration.

Local realignment is important for insertion and deletion sensitivity. While the modified Smith-Waterman alignment is a standard utility for alignments, it is computational intensive. In the methods provided herein, a modified Smith-Waterman local realign is used which limits the realignment to regions of interest, e.g., a few selected genes of interest (e.g., BRCA1 and BRCA2 genes). This modification decreases the computational duration of the realigner target creation and local realign steps. This also leads to quicker and more efficient system as run times can be reduced.

Deduplication is important in order to minimize the effect of amplification bias. It functions to remove apparent PCR duplicates by flagging reads that have the same start and end points when aligned against the reference sequence. Because shearing is random, it is unlikely that a fragment would share both a start point and an end point by chance, so those fragments are removed from the downstream analysis (only the highest quality fragment of the apparently clonal group is retained). After deduplication, the allele balance of heterozygous variants tends to be close to the expected 50%, which is important for sensitivity (e.g., if it drops too low due to amplification bias, the variant might be missed) and zygosity detection (e.g. if it is too high, then it could be mistaken for homozygous). The upstream bench process described herein for the preparation of the samples by bait capture and deduplication in the bioinformatics process facilitates a desirable allele balance.

Variants are then called at every position in the regions of interest (coding exons +/-50 bases). In one implementation, the UnifiedGenotyper in GATK v 2.3-9 can be used. In one particular embodiment GATK is employed using discovery mode, emitting all sites, calling indels and point mutations, with a maximum alternate alleles setting of two, and an indel gap opening penalty of 30. Calling all variants at every position ensures that no variant calls are suppressed. Ensuring that no variant calls are suppressed is important to the present method. For example, if a variant call is suppressed, deletions can be missed, leading to less accurate results. After forcing a variant call at every position, the positions not having variants are filtered out and the remaining variants proceed to downstream processing.

Variant calling in GATK can be performed in discovery mode or in genotyping mode, where a list of variants are supplied to call presence or absence. In some embodiments, both types of variant calling can be performed and the results combined, in order to maximize sensitivity. Such methods can address issues observed with different amplification protocols in the preparation of the samples (e.g. where PCR duplicates could not be removed).

In specific embodiments of the present invention, a filter or threshold may accordingly be implemented to distinguish between sequence data showing an acceptable base quality and a non-acceptable base quality. The term "acceptable base quality" as used herein refers to a phred-like quality score of about 20 and higher. A phred-like quality score is a Q score which is -10 log₁₀(e) where e is the estimated probability of the base call being wrong. The method is typically used to measure the accuracy of sequencing data. Higher quality scores indicate a smaller probability that a base is called incorrectly. A quality score of 20 thus represents an error rate of 1 in 100, with a corresponding call accuracy of 99%. In further specific embodiments of the present invention a filter or threshold may be implemented to distinguish between sequence data showing an acceptable coverage and a non-acceptable coverage. The term "acceptable coverage" as used herein refers to a coverage of about 20× and above. Accordingly, the number of reads covering a base in an alignment is about 20, or more.

In further specific embodiments of the present invention, a filter or threshold may be implemented to distinguish between sequence data showing an acceptable high complexity of the surrounding region and a medium to low complexity of the surrounding region. The term "high complexity of the surrounding region" as used herein refers to the presence of repeated sequences stretches, e.g. the presence of repeated dimers, trimers, the presence of transposon remnants or repeated sequences derived from transposons etc.

In yet another specific embodiment of the present invention a filter or threshold may be implemented to distinguish between sequence data showing an acceptable length of mismatch and non-acceptable length of mismatch. The term "acceptable length of mismatch" as used herein refers to gaps that does not allow complete matching of a read to the reference sequence. A corresponding matching may be a continuous and non-continuous matching of about 70% and more.

### Binary alignment/Map format

Figure 6 is a block diagram of a computer system in accordance with an illustrative implementation. The computer system can be used for the sequence data analysis described above, including performance of one or more or all steps of the QSAP pipeline. The exemplary computing system **600** includes a bus **605** or other communication component for communicating information and a processor **610** or processing circuit coupled to the bus **605** for processing information. The computing system **600** can also include one or more processors **610** or processing circuits coupled to the bus for processing information. The computing system **600** also includes main memory **615,** such as a random access memory (RAM) or other dynamic storage device, coupled to the bus **605** for storing information, and instructions to be executed by the processor **610**. Main memory **615** can also be used for storing position information, temporary variables, or other intermediate information during execution of instructions by the processor **610.** The computing system **600** may further include a read only memory (ROM) **620** or other static storage device coupled to the bus **605** for storing static information and instructions for the processor **610.** A storage device **625,** such as a solid state device, magnetic disk or optical disk, is coupled to the bus **605** for persistently storing information and instructions.

The computing system **600** may be coupled via the bus **605** to a display **635,** such as a liquid crystal display, or active matrix display, for displaying information to a user. An input device **630,** such as a keyboard including alphanumeric and other keys, may be coupled to the bus **605** for communicating information and command selections to the processor **610.** In another implementation, the input device **630** has a touch screen display **635.** The input device **630** can include a cursor control, such as a mouse, a trackball, or cursor direction keys, for communicating direction information and command selections to the processor **610** and for controlling cursor movement on the display **635.**

According to various implementations, the processes described herein can be implemented by the computing system **600** in response to the processor **610** executing an arrangement of instructions contained in main memory **615.** Such instructions can be read into main memory **615** from another computer-readable medium, such as the storage device **625.** Execution of the arrangement of instructions contained in main memory **615** causes the computing system **600** to perform the illustrative processes described herein. One or more processors in a multi-processing arrangement may also be employed to execute the instructions contained in main memory **615**. In alternative implementations, hard-wired circuitry may be used in place of or in combination with software instructions to effect illustrative implementations. Thus, implementations are not limited to any specific combination of hardware circuitry and software.

Although an example computing system has been described in Figure 6, implementations described in this specification can be implemented in other types of digital electronic circuitry, or in computer software, firmware, or hardware, including the structures disclosed in this specification and their structural equivalents, or in combinations of one or more of them.

Implementations described in this specification can be implemented in digital electronic circuitry, or in computer software, firmware, or hardware, including the structures disclosed in this specification and their structural equivalents, or in combinations of one or more of them. The implementations described in this specification can be implemented as one or more computer programs, i.e., one or more modules of computer program instructions, encoded on one or more computer storage media for execution by, or to control the operation of, data processing apparatus. Alternatively or in addition, the program instructions can be encoded on an artificially-generated propagated signal, e.g., a machine-generated electrical, optical, or electromagnetic signal that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium can also be, or be included in, one or more separate components or media (e.g., multiple CDs, disks, or other storage devices). Accordingly, the computer storage medium is both tangible and non-transitory.

The operations described in this specification can be performed by a data processing apparatus on data stored on one or more computer-readable storage devices or received from other sources. The term "data processing apparatus" or "computing device" encompasses all kinds of apparatus, devices, and machines for processing data, including by way of example a programmable processor, a computer, a system on a chip, or multiple ones, or combinations of the foregoing. The apparatus can include special purpose logic circuitry, e.g., an FPGA (field programmable gate array) or an ASIC (application-specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers that are located at one site or distributed across multiple sites and interconnected by a communication network

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

### Data reporting/Output

Downstream of variant calling, a metrics report generator, e.g., QC Metric (e.g. number of reads, mean coverage, minimum coverage), can be used to calculate regional minimum coverage depths per sample (counting reads having a minimum of Q30 at each position in the region of interest), followed by result parsing and cataloging in a purpose-built database, where samples having minimum coverage below 20 reads (after removing duplicate reads) were flagged for repeat.

In some embodiments of the present invention, the sequence analysis pipeline as described herein above may be associated or connected to a diagnostic decision support system. A "diagnostic decision support system" as used herein refers to system comprising an input for providing a subject's sequence data and, in specific embodiments, optionally its functional readout, for example gene or non-coding RNA expression, or protein levels. In addition, the system comprises a program element or computer program or a software for assembly of nucleic acid sequence data comprising nucleic acid fragment reads into contiguous nucleotide sequence segments, which when being executed by a processor is adapted to carry out the steps of the method as defined herein above, and an output for outputting a subject's contiguous nucleotide sequence segment(s) variation, and a medium for storing the outputted information. Preferably, the outputted information is able to indicate the presence or absence of genomic modifications, more preferably the affliction of a subject by a disease or a predisposition for a disease.

An automated preliminary assessment can be performed on the variant call file data and retuned as an annotated file. The initial automated assessment can include variant molecular classification (e.g. synonymous, missense, nonsense, frameshift), comprehensive clinical evidence (largely curated by scientists from peer- reviewed literature) and evidence-based clinical decision support that assists the initial classification of the observed variants. The preliminary automated assessment improves the turnaround time and maximizes the information used for the final clinical assessment of variants. The assessment software can be configured using pre-defined scoring and classification rules, including American College of Medical Genetics recommended guidelines and/or a compilation of evidence including the most recent literature. The assessment software can provide reviewers with direct access to the relevant literature for variants as well as providing transparency to how the automated assessments were derived to facilitate review of primary data. Final classification can also be perform using manual review through a web interface, additional locus-specific databases and confirmatory queries.

Sequencing data or assessment analyses can be reported to a certified laboratory, to a physician, or directly to the patient. Customized visualization can used to manually review the results for nomination to the clinical report. In yet another embodiment of the present invention, the diagnostic decision support system may be an electronic picture/data archiving and communication system.

The disease or disorder which may be detected or diagnosed or prognosticated according to the present invention may be any detectable disease known to the person skilled in the art. In a preferred embodiment, said disease may be a genetic disease or disorder, in particular a disorder, which can be detected on the basis of genomic sequence data Such disorders include, but are not limited to, the disorders mentioned, for example, in suitable scientific literature, clinical or medical publications, qualified textbooks, public information repositories, internet resources or databases, in particular one or more of those mentioned in http://en.wikipedia.org/wiki/List_of_genetic_disorders.

In a particularly preferred embodiment of the present invention said disease is a cancerous disease, e.g. any cancerous disease or tumor known to the person skilled in the art. In particular embodiments, the disease is breast cancer, ovarian cancer, or prostate cancer.

In a specific embodiment, said diagnostic decision support system may be a molecular oncology decision making workstation. The decision making workstation may preferably be used for deciding on the initiation and/or continuation of a cancer therapy for a subject. Further envisaged are similar decision making workstation for different disease types, e.g. for any of the diseases as mentioned herein above.

### EXAMPLES

### Example 1

This disclosure describes the development and validation of a rapid, high-throughput sequencing assay for the detection of BRCA1 and BRCA2 variants suitable for the clinical laboratory. Results from the initial 1006 clinical samples tested in duplicate with the MiSeq/QSAP combination showed no discrepant variant calls. In one implementation, an NGS-based assay using bait tile exon capture for detection of BRCA1/2 variants in a reference laboratory. During testing, two different NGS platforms were employed: the Illumina MiSeq System and the Life Technologies Ion Torrent Personal Genome Machine. As explained below, results from the first 521 clinical samples were obtained using both NGS platforms, and an additional 1006 results were obtained using duplicate MiSeq runs.

Figure 1 illustrates the general overview of the NGS assay for detection of BRCA1 and BRCA2 variants. The description below describes one implementation of the invention.

### DNA Samples

DNA samples from cell lines with known deleterious variants in BRCA1 (n=21; Table 1) or BRCA2 (n=6; Table 1) were purchased from the Coriell Mutant Cell Repository (Camden, NJ). Theses reference samples contained both pathogenic and nonpathogenic variants. In one implementation, blood samples from 67 unaffected individuals previously untested for BRCA mutations were obtained. Sanger sequencing was performed to determine the presence of BRCA1 or BRCA2 sequence variations. In this implementation, 352 benign variants were identified in the volunteer population and were used in the technical validation.

### DNA Preparation

Genomic DNA from peripheral blood cells was isolated in 96-well microtiter plates using a Roche Magnapure^{™} system from Roche Molecular Systems (Indianapolis, IN) per the manufacturer's instructions. Genomic DNA was randomly sheared to an average size of 250 base pairs using adaptive focused acoustics technology (E220 Focused Ultra-Sonicator, Covaris Inc., Woburn, MA) according to the manufacturer's instructions.

### Concentration via SPRI Beads and PEG/Sodium Chloride Mix

After shearing, the DNA was concentrated 2-fold and DNA molecules with inadequate sizes were removed. This was accomplished with SPRI (solid-phase reversible immobilization) beads (AMPure Beads, Agencourt, Beverley, MA). The beads were suspended in a solution of polyethylene glycol (PEG), EDTA.

### DNA End Repair

The ends of the DNA molecules were repaired prior to adaptor ligation. This was accomplished using a DNA polymerase that has both 5' to 3' polymerase activity and 3' to 5' exonuclease activity, thereby filling in 5' overhangs and removing 3' overhangs to generate blunt ends. In addition, the 5' end of the DNA fragments were also phosphorylated in this process.

### Adaptor Ligation and Nick Repair

Each 5' adaptor contains a unique molecular identification (MID) sequence (barcode) that is used to identify individual DNA samples. In addition, it contains a portion of the P5 sequence. The 3' adaptor is universal to all specimens and contains a portion of the P7 MiSeq sequence. Neither adaptor is 5' phosphorylated. A short complimentary oligonucleotide for each of the adaptors can also be included in the ligation reaction to ensure that the adaptors are only ligated to the DNA fragments and not to themselves. During ligation the molar ratios of the two adaptors are equal to each other but are in excess in comparison to the fragmented DNA. Following this procedure, approximately half of all ligation products are the preferred species, namely: 5'-(P5)- MID-BRCA_GeneDNA-universal (P7)-3'. The samples were cleaned using SPRI beads as described above, and the nicks at the ligation site were repaired by a DNA polymerase. The polymerase adds nucleotides at the nick site creating a primer binding site for PCR amplification.

### Pre-hybridization Amplification

In order to increase the ratio of adapted DNA fragments, non-allele specific PCR was performed. The primers used were complementary to the 5' and the 3' adaptor sequences.

### Target Enrichment Through Exon Capture

All bar coded patient DNA fragments were pooled to create a "library," and were added to a hybridization reaction mixture and incubated for 12 hours at 65°C. This mixture contained the biotinylated RNA baits. The baits were complimentary to BRCA1 and BRCA2 genes (exonic regions and splice junction sites, and a selected intronic region) to allow hybridization to the appropriate patient DNA fragments. After the hybridization, the library was combined with streptavidin coated beats to adsorb the biotinylated RNA baits. The library-RNA bait hybrids were washed at 70°C to remove the non-BRCA DNA.

### Second Nonspecific Amplification

Additional sequences required for either the Illumina MiSeq^{™} (Illumina, San Diego, CA) or Ion Torrent^{™} Personal Gene Machine (PGM) (Life Technologies, Grand Island, NY) sequencing platforms were added to the 5' and 3' adaptors using fusion primers. The DNA library was divided into two halves. One half was amplified with fusion primers (P5 and P7 sequences) that have a portion complementary to the 5' and 3' adaptors and add additional sequences for MiSeq sequencing and the other half was amplified with a set of primers (P1 and A sequences) that add additional sequences for PGM sequencing.

### Quantification of DNA Concentration by Qubit

The high sensitivity Qubit kit (Life Technologies), which uses an intercalating dye based method, was used to quantify DNA.

### NGS Sequencing

The prepared library was diluted so that amplification generated well-separated clusters of identical products from a single DNA molecule (i.e. clonal amplification). The MiSeq and PGM NGS protocols were performed according to the manufacturer's protocols.

### MiSeq

The single-stranded library was loaded into well 21 of the MiSeq sequencing cartridge. The instrument flushed the library through the flow cell where it hybridized to the antisense P5 and P7 oligonucleotides that are complimentary to the adaptors on the library. The library was diluted so that amplification generated well-separated clusters of identical products from a single DNA molecule (clonal amplification). This was accomplished by isothermal bridge amplification. Fluorophore-labeled nucleotide triphosphates were applied to the flow cell and then excited by a laser. The emission spectra was recorded by the MiSeq, and then the nucleotide blocker, which inhibited further synthesis, was cleaved, allowing for addition of the next nucleotide triphosphate. In this manner, fragments were sequenced.

### Ion Torrent^{™} PGM

The PGM uses emulsion PCR, the amplification inside of tiny water droplets floating in oil. Emulsion PCR is performed to get many copies of a single DNA molecule onto a single sequencing "bead" (i.e. clonal amplification). These beads are then used to generate the sequence. The beads used in the amplification solution are covered with covalently bound oligonucleotides that are antisense to the P1 sequence of the library. Micro-chambers are created by the Ion Torrent One Touch Instrument 2 (OT2, Life Technologies, Grand Island, NY), which carries out clonal amplification.

The strand of DNA that results from extension of the anti-sense P1 oligonucleotide is then hybridized at its 3' end with a sequencing primer that binds at the anti-sense A oligonucleotide. DNA polymerase is added to the beads and then the beads are deposited into tiny pores on the surface of a computer chip-like surface. Each of the four dNTPs is then sequentially flowed in excess over the surface of the chip. The DNA polymerase extends the growing strand when the required nucleotide is made available. Whenever a nucleotide is added, a hydrogen molecule is released resulting in a pH change in the pore containing the sequencing bead. The magnitude of the pH change is approximately equal to the number of nucleotides incorporated and is detected and measured along with which of the four nucleotides that flowed through.

### Bioinformatics Processing

Following the sequencing reaction, sequence alignment and allele assignment was performed. Initially, for the MiSeq the MiSeq Reporter^{™} software supplied with the instrument was used. This process, however, did not consistently identify deletions larger than 9 bp. To identify deletions larger than 9 bp, the following workflow, called QSAP, was used. QSAP is a bioinformatics pipeline. The QSAP is the specialized portion of the overall workflow that integrates open source, in-house developed and licensed modules for sequence analysis. This analysis pipeline, using a high performance computing infrastructure, includes the Burrows Wheeler Aligner (BWA) for mapping to the hg19/GRCh37.1 reference genome and Queue with the Genome Analysis Tool Kit (GATK) for deduplication, modified Smith-Waterman local realignment, and variant calling. The pipeline is designed to maximize the accuracy of variant calls, reduce time of analysis and permit ready access to sample Binary alignment/Map format (BAM) and Variant Call Format (VCF) files.

Specifically, following copying sample FASTQ nucleotide sequence files from the MiSeq instrument (demultiplexed using index reads on-instrument), the sample FASTQ files were filtered to remove reads flagged as failing a vendor quality filter. This procedure can be performed using a sequence sorting utility, such as, for example, SAMtools (Sequence Alignment/Map tools). The adapter and Molecular Identifier (MID) tag sequences (i.e. identifier sequences ligated to the sample's sequence during the nucleic acid sample preparation process) are trimmed. In one implementation, this process is done using a FASTQ processing utility, such as fastq-mcf, which scans a sequence file for adapters, and, based on a log-scaled threshold, determines a set of clipping parameters and performs clipping.

Following trimming, the reads were mapped to genomic reference, sorted and indexed. This can be done using a Burrows-Wheeler Aligner (e.g. version 0.7.5a-r405) (e.g. using the command: bwa mem -M -t 2) and SAMtools. Read groups can then added. The read groups can be added using Picard, which comprises Java-based command-line utilities for manipulating SAM files. The next steps of the process involved pipeline realigner target creation, local realign, and flagging of duplicate reads. In some implementations, Queue can be used for these steps. In one implementation, the Queue pipeline was based on the DataProcessingPipeline scala script included in the Queue v.2.3-9 distribution. The following modifications were made in this implementation: customized the memory and thread usage parameters to fit the computing platform, used the modified Smith-Waterman local realign option, removed the base quality score recalibration step, and limited the Queue analysis and BAM output to the regions of interest (coding exons +/- 50 bases) padded by an additional 150 bases. Variants were then called at every position in the regions of interest (coding exons +/- 50 bases). In one implementation, the UnifiedGenotyper in GATK v 2.3-9 was used, using discovery mode, emitting all sites, calling indels and point mutations, with a maximum alternate alleles setting of two, and an indel gap opening penalty of 30.

For the PGM data, bioinformatics analyses were performed using the Torrent Suite^{™} software supplied with the instrument.

Downstream of variant calling, a metrics report generator, e.g., QC Metric (e.g. number of reads, mean coverage, minimum coverage), can be used to calculate regional minimum coverage depths per sample (counting reads having a minimum of Q30 at each position in the region of interest), followed by result parsing and cataloging in a purpose-built database, where samples having minimum coverage below 20 reads (after removing duplicate reads) were flagged for repeat.

In some embodiments, the BRCA1/2 advanced sequencing bioinformatics modular workflow manages the sequence information from Illumina MiSeq FASTQ files to final reporting to a certified laboratory, e.g. CLIA (Clinical Laboratory Improvement Amendments) or CAP (College of American Pathologist) certified laboratory. In some embodiments, the workflow uses customized visualization to manually review the results for nomination to the clinical report.

In some embodiments, the de-identified VCF files are subjected to automated preliminary assessment, and an annotated file is generated. The initial automated assessment leverages variant molecular classification (e.g. synonymous, missense, nonsense, frameshift), comprehensive clinical evidence (largely curated by scientists from peer- reviewed literature) and provides evidence-based clinical decision support that assists the initial classification of the observed variants. The preliminary automated assessment improves the turnaround time and maximizes the information used for the final clinical assessment of variants. The automated assessment has two additional fundamental features. First, the assessment was configured using Quest Diagnostics pre-defined scoring and classification rules with American College of Medical Genetics recommended guidelines being the central advisement combined with a compilation of evidence including the most recent literature. Second, the software provides reviewers with direct access to the relevant literature for variants as well as providing transparency to how the automated assessments were derived to facilitate review of primary data. Subsequent manual review through Ingenuity's VCS web interface, additional locus-specific databases and confirmatory queries were carried out to complete the final classification. For the PGM data, bioinformatics analyses were performed using the Torrent Suite^{™} software supplied with the instrument.

### Variant assessment

Variant assessment is performed manually by a team of variant scientists (VS) according to the guidelines of the American College of Medical Genetics. VCF files are analyzed by the software program Alamut that provides genomic coordinates and SIFT *in vitro* functional analysis. The VS rechecks the quality metrics of the individual run and, if the run passes QC, proceeds to the assessment. At this point the deletion/duplication results from the MLPA reactions are also reviewed.

The VS then reviews the called variants to ensure concordance with the IGV data. If variant identification is accurate, the variants are loaded from Alamut HT into a proprietary database called QuestIQ. The following fields are automatically filled by the Alamut HT software interface: Gene|Variant, Variant ID, Ref Seq, DNA level, Mutation type, Code Interpretation, PUC, Gene Code, Exon, Nucleotide, Change, Codon, Amino Acid, dbSNP rs#, dbSNP link, SIFT, Species conservation, Link to VUS analysis text, link to Splicing Report, and MolGen accession numbers.

The VS then searches for further information using gene specific databases, e.g., UMD, BIC, LOVD, IARC, ClinVar, ARUP, kConFab, HGMD, InSIGHT. This is followed by assessing the variant frequency using ESP and dbSNP. If applicable, post translation predictive databases are used such as NetPhosk, NetPhos, ScanSite: S, T, Y phosphorylation predictions, Yin o Yang: O-linked GlcNac prediction. Splicing predictions are made using linked software in Alamut HC, using the RefSeq database. The functional predictive programs SIFT and PolyPhen2 are then used.

Subsequently, a manual literature search is performed to determine if there is further supporting data on the particular variant using a Google search through Alamut, PubMed, ScienceDirect, and BioMed Central All relevant results are entered into the IQDB database.

The final variant classification is made according to the ACMG guidelines, and the result entered into the IQDB database. Classifications are scored as Benign, Likely Benign, VUS, Likely Pathogenic, Pathogenic. This result is then passed to a director for secondary review and report writing.

### Results

### Assay Development

During assay development, the MiSeq sequencing system using the supplied MiSeq Reporter software was unable to identify 2 of the pathological BRCA1 variants in tested Coriell samples. Both variants were deletions of >9 bp: the 40-bp deletion c. 1175_1214del40 and the 10-bp deletion c.3481_3491del10. These were the only deletions of >9 bp in these samples. The QSAP workflow described above, however, can be used for alignment and allele assignment (QSAP) as described above. Figure 3 shows the alignment representation for the sample containing a 40-bp deletion. This deleterious mutation was identified by the PGM/Torrent Suite software but not the MiSeq/MiSeq Reporter software. However, the deletion was clearly identified when using MiSeq with QSAP software (Figure 3). Similar findings were seen for the 10-bp deletion, with MiSeq/MiSeq reporter consistently missing the deletion and the PGM/Torrent Suite and MiSeq/QSAP always identifying the deletion (data not shown). The MiSeq/QSAP and the PGM/Torrent Suite combinations both showed 100% sensitivity for the BRCA1 and BRCA2 variants in the validation set. Technical validation using both platforms was performed.

Since NGS sequencing errors can result from PCR or clonal amplification errors, QC metric was developed to overcome these potential problems, taking advantage of the fact that random shearing leads to library clones that have different starting and ending positions. Therefore the bioinformatics analyses were able to differentiate among reads from the same versus different library clones This enabled us to develop a minimum QC metric, whereby each targeted base must have high quality sequence from a minimum of 20 unique clones. Typically however, an "average depth of read" of 335 unique reads was achieved.

### Technical Validation: Intra-assay Precision

Intra-assay precision was established by analyzing the DNA extracted from three blood samples, in five replicates on each platform. Each sample had at least one BRCA1 or BRCA2 variant. All variants identified in each sample were 100% concordant within the five replicates when detected on the MiSeq/QSAP combination. However, the PGM/Torrent Suite platform exhibited a low level of random sequencing errors. Overall intra-assay concordance on the PGM instrument was only 96.2%. In one sample, a single base insertion was detected in the fifth replicate that was not detected in the other replicates. In addition, a benign variant was not identified in the fourth replicate. In another sample, the fifth replicate contained 4 SNPs that were not present in any of the other replicates, and one SNP was called in replicate 3 that was not called elsewhere.

In comparing the MiSeq/QSAP allele calls to the PGM/Torrent Suite calls, there were several discordances. One sample showed discordance at 2 SNP sites between the 2 platforms: PGM/Torrent Suite called an A insertion at position 32906554 on chromosome 13 in one of the replicates that was not identified in the remainder of the PGM/Torrent Suite replicates or on the MiSeq/QSAP platform. The PGM/Torrent Suite also did not detect rs1799949 at position 41245466 on chromosome 17 in one replicate. As a result, intra-specimen concordance was 88% between the 2 platforms for this specimen. The second sample was concordant on all variant calls from both platforms, resulting in a concordance of 100% between platforms. The third sample was discordant at 5 of 11 SNPs due to sequencing errors on PGM/Torrent Suite (1 of 5 replicates for each SNP), for a concordance rate of only 64%.

### Technical Validation: Inter-assay Precision

DNA from remnant laboratory samples from 67 presumably unaffected individuals, plus the 27 DNA specimens from Coriell, were analyzed in 3 replication set-ups. Two negative controls (a quality control blank [QCB]) and a no-template control [NTC]) were also included in each run. Libraries prepared for each run were detected on both the MiSeq/QSAP and PGM/Torrent Suite platforms. All variants detected in 3 replication runs from both the PGM and MiSeq instruments were verified by manual review using IGV (version 2.3.14). Specimens failing in 2 or more of the replication runs were excluded from the inter-assay variability assessment.

Fewer verified variants were detected on the PGM/Torrent Suite platform than on the MiSeq/QSAP platform, owing to higher assay failure rate on the former (Table 2). The inter-assay precision was 96.7% for the PGM/Torrent Suite and 99.4% for the MiSeq/QSAP (Table 2). Of note, discrepant calls detected among the 3 replicates on the MiSeq/QSAP platform represented false-positive results; most came from a single sample on a single replicate, likely indicating a problem with sample preparation or well contamination.

**Table 2. Inter-Assay Concordance of Variant Calls vs. Sanger**

| | PGM/Torrent Suite | MiSeq/QSAP |
|---|---|---|
| Concordant Calls | 1550 | 2188 |
| Discrepant Calls | 53 | 13 |
| Total Calls | 1603 | 2201 |
| % Concordance | 96.7% | 99.4% |

| | | |
|---|---|---|
| Samples analyzed included 27 control DNA specimens with known deleterious mutations and 67 specimens from volunteers with a total of 352 benign variants. | | |

### Failed Specimens

Specimen failure was defined as failure to achieve an average coverage depth of >40x at any exon. For the MiSeq instrument, there were no failures in replications 1 and 2 and 8 specimen failures in replication 3. Therefore, the overall failure rate was 8.5% (8/94) for replication 3, or 2.8% (8/282) overall. For the PGM, the failure rate was 9.6% (9/94) for replication 1, 13.8% (13/94) for replication 2, and 26.6% (25/94) for replication 3; the overall failure rate was 16.7% (47/282). All the failed specimens with low coverage were among the control specimens from consented subjects, possibly reflecting higher DNA quality in the Coriell DNA samples.

Four specimens failed in replication 3 on the MiSeq/QSAP platform and in all 3 replication runs on the PGM/Torrent Suite platform. This finding suggests specimen quality issues, although the same specimens were successfully sequenced for all regions in replications 1 and 2 on the MiSeq/QSAP platform. All the specimens that failed on the MiSeq/QSAP platform were from replication 3. The failure rate on the PGM/Torrent Suite platform was also highest for replication 3. This points to a sample preparation issue for that replication, as the sample libraries for both platforms were prepared together up to and through the hybrid capture step.

### Inter-platform Concordance

As there were more failures in the PGM runs than in the MiSeq runs, only a subset of discrepant variant calls from the MiSeq/QSAP platform with calls from the PGM/Torrent Suite platform was verified. All 8 discrepant variant calls from MiSeq/QSAP were from replication 2. Four of the 8 were similarly observed in replication 2 on the PGM/Torrent Suite platform. In addition, 5 of the 8 variant calls were observed in a single sample.

### Analytic Sensitivity: Detection Limits

### Limit of Blank (LOB)

The NTC and the QCB bar coded specimens were carried throughout the assay and handled identically to all other specimens. The number of reads mapped to the hg19 genome sequence was compared to the average aligned reads per sample. For the MiSeq/QSAP platform in replication 1, no reads were mapped to the human genome for either the NTC or the QCB. In replication 2, the QC blank had no reads but the NTC had 1,486 reads. This totaled 1.1% of the average number of reads on the plate and was well below the 20% threshold for an allele call. In replication 3, the NTC had 46 reads, representing 0.044% of the average number of reads in plate 1.

For the PGM/Torrent Suite platform, the NTC and QCB demonstrated 0.255% and 0.029% aligned reads in plate 1. In plate 2, the NTC had 9.2% of the average aligned reads while the QC blank had zero. The values for the 3rd replication plate were 0.268% and 0 063% for the NTC and QCB, respectively. The NTC and QCB demonstrated an acceptably low overall number of aligned reads on both platforms. The aligned reads were either not detectable or well below our cutoff threshold of 20% for variant calls.

### Limit of Detection (LOD)

The LOD was defined as the lowest DNA concentration (ng/µL) at which the average read depth over the exonic region was maintained at ≥40 reads per base. To determine the LOD, the following experiments can be used. Two Coriell DNA samples, GM14094 and GM14096, and a single random DNA sample chosen from the 67 control individuals lacking pathogenic BRCA were serially diluted. On the MiSeq/QSAP platform, the control DNAs failed to achieve the required average read/coverage depth at 5 ng/µL demonstrating that the minimal sample input (LOD) for the MiSeq/QSAP platform must be greater than 5 ng/µL (all shearing reactions were carried out in 80 µL volumes). In addition, all variants were consistently called (i.e., 100% concordant) at each concentration for the 3 specimens above this lower limit. On the PGM/Torrent Suite platform, the samples failed at 5 ng/µL, demonstrating that the minimal sample input must also be greater than 5 ng/µL (400 ng of DNA). On both platforms, the 40-bp and 11-bp deletion mutations were successfully detected at all concentrations. However, only 99.96% of the called variants were concordant for the non-Coriell control sample. At 15 ng/µL, an insertion was called in the control DNA using the PGM/Torrent Suite platform that was not present in any of the other concentrations and was likely due to a sequencing error.

### Accuracy

The 27 DNA specimens obtained from Coriell were included in this validation study, in 3 separate runs, on both MiSeq/QSAP and PGM/Torrent Suite platforms. All previously known BRCA1 and BRCA2 variants in the specimens were successfully detected by both platforms and in all three validation runs (i.e., 100% accuracy for cancer-associated mutations). In addition, the overall accuracy of variant calls for the 352 benign sequence changes detected in the 67 control samples was determined. There was only one missed call on the MiSeq/QSAP platform, which was due to low read depth (coverage). This error could have been avoided by adjusting the minimum depth requirement in our QC metric as this was implemented prior to going live with the assay. The PGM/Torrent Suite platform yielded two false-positive calls, one sequencing error, and 37 missed variant calls, most of which were observed in only one of the three validation runs. However, there were 4 variants not called by Ion Reporter, which were detected by manual review of the alignment software. Overall, the error rate was <0.1% (1/1056) for the MiSeq/QSAP platform and 3.7% (39/1056) for the PGM/Torrent Suite platform. With the adjusted QC parameters, the MiSeq/QSAP combination had 100% sensitivity and nearly 100% specificity. With manual review of all positive samples, the MiSeq/QSAP combination also achieved 100% specificity.

### The First 521 Clinical Samples

For the initial clinical test release, mutation analyses were performed using both the MiSeq/QSAP platform and the PGM/Torrent Suite platform variant calling software. For samples with discrepant results on the 2 platforms, either the cases were manually reviewed to determine the cause of the discrepancy or the samples were retested for confirmation. There were 35 discrepancies in the first 521 reported cases, with 34 due to PGM/Torrent Suite errors. The single MiSeq/QSAP platform sequencing error was a false-negative result for a benign polymorphism. Manual review of the alignment revealed that this was due to a combination of strand bias (19% variant) and low coverage. The QC parameters can then be adjusted to take advantage of the fact that random shearing allows the filtering of duplicate reads. The QC acceptance metric requires that each base in each assay be analyzed from at least 20 independent reads. This typically resulted in an average depth ranging from several hundred to a few thousand. Using adjusted QC parameters, the MiSeq/QSAP combination had 100% sensitivity and 100% specificity. For all positive cases, the alignments are manually reviewed as a further quality measure.

After making these adjustments to the QC metrics, the MiSeq/QSAP platform has made no further errors in more than 500 consecutive analyses. However, the PGM/Torrent Suite combination suffered 2 false-negative results for pathogenic BRCA1 variants: a 10-base pair insertion and a 64bp deletion. Both of these pathogenic variants were detected with the MiSeq/QSAP platform. Figure 4 shows the QSAP alignment for this 64-bp deletion. Following this observation the use of the PGM/Torrent Suite platform can be discontinued. During testing, to be certain that our new quality metric would ensure the identification of all variants, all MiSeq/QSAP analyses were performed in duplicate. Duplication would ensure that any false-positives or false- negatives due to strand bias, low coverage, or library creation would be detected. In 1006 consecutive duplicate MiSeq/QSAP runs with more than 5000 variants detected, there were no discrepant results between duplicate analyses (data not shown). Therefore, the duplicate run is not required.

An advantage of NGS platforms over standard Sanger sequencing is their ability to determine if 2 SNPs are cis or trans in orientation. If two variants are captured in a single read (in this case less than 250 bases), they are revealed as being in cis. If they are captured on separate reads, then they are revealed to be in trans. Figure 5 shows an individual who has two point mutations in cis. Two such linked variants in the first 521 clinical samples have been seen. In addition, in routine operations, the MiSeq/QSAP platform was considerably more robust than the PGM/Torrent Suite platform. Accordingly, the PGM/Torrent Suite platform test can be discontinued and performing duplicate MiSeq/QSAP runs for each case can be done to determine if there are any potential problems with false-positives or false-negatives due to library formation.

There was a single discrepancy between the duplicate MiSeq runs in noted in the initial 100 duplicate samples. This was a benign polymorphism that was detected in one run, but not detected in the second run. Examination of the sequencing data revealed that there was significant strand bias in the second sequencing run leading to 19% a variant frequency for the variant. Our QC cut-off at the time was 20%. In order to prevent reoccurrence of this error, the QC metric for variant calling was modified. The random fragmentation of the DNA causes each individual DNA fragment to have a unique starting and ending nucleotide. This allows the bioinformatics to uniquely identify the clones sequenced. The QC metric was adjusted to require that each base in every coding exon and 50 base pairs into the exon be sequenced from at least 20 different clones. This eliminates errors due to sequencing bias, since reads from an overrepresented clone with be ignored, and by requiring a minimum number of clones to be sequenced, heterozygotes have close to a 50% representation. After making this adjustment, no discrepancies were found in more than 1006 consecutive analyses.

The data demonstrate that neither the Illumina MiSeq sequencer with the supplied MiSeq Reporter software nor the Life Technologies PGM with the supplied Torrent Suite software are suitable for clinical laboratory sequencing of BRCA1 and BRCA2. The MiSeq system's inability to detect insertions and deletions larger than 9 bp makes it unacceptable for BRCA testing, as many of the described deleterious mutations are in that size range. Similarly, the inability of the PGM with Torrent Suite software to detect a 10-base pair insertion and 64-bp deletion disqualifies that platform from clinical BRCA testing. However, by combining random shearing with bait tile capture, the MiSeq platform with the bioinformatics of the QSAP alignment and allele calling software, and our quality metrics, the disclosed assay has 100% sensitivity and specificity for BRCA1 and BRCA2 sequence variations in our technical validation series. Real-world performance may not reach this level of precision.

The use of NGS with bait tile exon capture offers several advantages. First, bait tile exon capture prior to NGS decreases the likelihood of false-negative results due to allele drop-out, which may occur with PCR-based methods when polymorphisms are present in amplification or sequencing primer sequences. Second, with 5x redundant tiling, each exon is captured by multiple baits, further reducing the chance of a false-negative result due to individual sequence variation. A third advantage of bait tile capture versus PCR-based target enrichment methods is the avoidance of false-positive results due to amplicon bias in PCR or library formation. If a base substitution error occurs in an early PCR or library amplification cycle, the error will be propagated and result in a mixed population prior to sequencing. If an error occurs in a single amplicon, and the amplicon is preferentially sequenced, this can result in a false-positive result. With the bait tile capture approach, genomic DNA is randomly sheared to fragments of approximately 250 bp prior to bait tile capture. Library formation occurs after capture. Thus, each fragment has different 5 and 3 prime termini, and the sequence alignment software can detect if 2 reads are generated from the same fragment. Filters can be set to only accept reads from unique fragments, thereby eliminating the possibility of sequencing errors due to early PCR or library amplification errors. The selected quality control metrics require reads from at least 20 different clones, minimizing the risk of false-positive sequencing results in NGS.

Relative to Sanger sequencing, NGS also has the advantage of detecting the phase of SNPs within approximately 250 bp (i.e., the length of sheared genomic DNA fragments). Since this technology sequences a single molecule, 2 SNPs that are in cis orientation will appear together in the same read; if the orientation is trans, the 2 SNPs will appear in separate reads. Sanger sequencing cannot differentiate between cis and trans orientation without resorting to family studies.

In conclusion, described herein is the development and validation of a rapid, high-throughput sequencing assay for the detection of BRCA1 and BRCA2 variants suitable for the clinical laboratory. Results from the initial 1006 clinical samples tested in duplicate with the MiSeq/QSAP combination showed no discrepant variant calls.

While this specification contains many specific implementation details, these should not be construed as limitations on the scope of any inventions or of what may be claimed, but rather as descriptions of features specific to particular implementations of particular inventions. Certain features described in this specification in the context of separate implementations can also be implemented in combination in a single implementation. Conversely, various features described in the context of a single implementation can also be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings and tables in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems can generally be integrated in a single software product or packaged into multiple software products.

Thus, particular implementations of the invention have been described. Other implementations are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

### Preferred Items:

1. A method for determining the presence of a variant in one or more genes in a subject comprising:
   (a) obtaining raw sequencing data pertaining to the subject from a nucleic acid sequencer;
   (b) removing low quality reads from the raw sequencing data that fail a quality filter;
   (c) trimming adapter and/or molecular identification (MID) sequences from the filtered raw sequencing data;
   (d) mapping the filtered raw sequencing data to a genomic reference sequence to generate mapped reads;
   (e) sorting and indexing the mapped reads;
   (f) adding read groups to a data file to generate a processed sequence file;
   (g) creating realigner targets;
   (h) performing local realignment of the processed sequence file to generate a re-aligned sequence file;
   (i) removing duplicate reads from the re-aligned sequence file;
   (j) analyzing coding regions of interest; and
      (i) generating a report that identifies whether the variant is present based on the analysis in step (j),
   wherein steps (g) and (h) are performed using a modified genomic alignment utility limited to nucleic acid regions containing the one or more genes of interest.
2. The method of item 1, further comprising performing the nucleic acid sequencing reaction on the nucleic acid sample from the subject using a nucleic acid sequencer to generate the raw sequencing data of step (a).
3. The method of item 1, wherein analyzing coding regions of interest comprises calling variants at every position in the regions of interest.
4. The method of item 3, wherein the regions of interest are padded by an additional 150 bases.
5. The method of item 3, wherein variant calling is performed with a modified GATK variant caller.
6. The method of item 1, wherein mapping the reads to a genomic reference sequence is performed with a Burrows Wheeler Aligner (BWA).
7. The method of item 1, wherein mapping the reads to a genomic reference sequence does not comprise soft clipping.
8. The method of item 1, wherein the genomic reference sequence is GRCh37.1 human genome reference.
9. The method of item 1, wherein the sequencing method comprises emulsion PCR (emPCR), rolling circle amplification, or solid-phase amplification.
10. The method of item 1, wherein the solid-phase amplification is clonal bridge amplification.
11. The method of item 1, wherein the nucleic acid is extracted from a biological sample from a subject.
12. The method of item 11, wherein the biological sample is a fluid or tissue sample.
13. The method of item 11, wherein the biological sample is a blood sample.
14. The method of item 1, wherein the nucleic acid is genomic DNA.
15. The method of item 1, wherein the nucleic acid is cDNA reversed transcribed from mRNA.
16. The method of item 1, wherein the nucleic acid is prepared prior to sequencing by performing one or more of the following methods:
   (a) shearing the nucleic acid;
   (b) concentrating the nucleic acid sample;
   (c) size selecting the nucleic acid molecule in a sheared nucleic acid sample;
   (d) repairing ends of the nucleic acid molecules in the sample using a DNA polymerase,
   (e) attaching one or more adapter sequences;
   (f) amplifying nucleic acids to increase the proportion of nucleic acids having an attached adapter sequence;
   (g) enriching the nucleic acid sample for one or more genes of interest; and/or
   (h) quantification of the nucleic acid sample primer immediately prior to sequencing.
17. The method of item 16, wherein the one or more adapter sequences comprises nucleic acid sequences for priming the sequencing reaction and/or a nucleic acid amplification reaction.
18. The method of item 16, wherein the one or more adapter sequences comprises a molecular identification (MID) tag.
19. The method of item 16, wherein enriching the nucleic acid sample for one or more genes of interest comprises exon capture using one or more biotinylated RNA baits.
20. The method of item 19, wherein the biotinylated RNA baits are specific for exonic regions, splice junction sites, or intronic region or one or more genes of interest.
21. The method of item 19, wherein the one or more biotinylated RNA baits are specific for a *BRCA1* gene and/or a *BRCA2* gene.
22. The method of item 1, wherein the subject is a mammal.
23. The method of item 1, wherein the subject is a human patient.
24. The method of item 1, wherein the subject is a human suspected of having cancer or suspected of being at risk of developing a cancer.
25. The method of item 24, wherein the cancer is a breast or ovarian cancer.
26. The method of any of items 25, wherein one or more variants in a gene associated with a cancer are determined.
27. The method of any of items 1-26, wherein one or more variants in the *BRCA1* gene or *BRCA2* gene are determined.
28. The method of any of items 1-27, wherein one or more variants is selected from the variants listed in Table 1.
29. The method of any of items 1-28, further comprising confirming the presence of the variant by Sanger sequencing.
26. A system comprising:
   one or more electronic processors configured to:
      (a) remove low quality reads from the raw sequencing data that fail a quality filter;
      (b) trim adapter and/or molecular identification (MID) sequences from the filtered raw sequencing data;
      (c) map the filtered raw sequencing data to a genomic reference sequence to generate mapped reads;
      (d) sort and index the mapped reads;
      (e) add read groups to a data file to generate a processed sequence file;
      (f) create realigner targets;
      (g) perform local realignment of the processed sequence file to generate a re-aligned sequence file;
      (h) remove of duplicate reads from the re-aligned sequence file; and
      (i) analyze coding regions of interest.
30. The method of item 29, wherein analyzing coding regions of interest comprises calling variants at every position in the regions of interest.
31. The method of item 30, wherein the regions of interest are padded by an additional 150 bases.
32. The method of item 30, wherein variant calling is performed with a modified GATK variant caller.
33. The method of item 29, wherein mapping the reads to a genomic reference sequence is performed with a Burrows Wheeler Aligner (BWA).
34. The method of item 29, wherein mapping the reads to a genomic reference sequence does not comprise soft clipping.
35. The method of item 29, wherein the genomic reference sequence is GRCh37.1 human genome reference.
36. A non-transitory computer-readable medium having instructions stored thereon, the instructions comprising:
   (a) instructions to remove low quality reads that fail a quality filter;
   (b) instructions to trim adapter and MID sequences from the filtered raw sequencing data;
   (c) instructions to map the filtered raw sequencing data to a genomic reference sequence to generate mapped reads;
   (d) instructions to sort and index the mapped reads;
   (e) instructions to add read groups to a data file to generate a processed sequence file;
   (f) instructions to create realigner targets;
   (g) instructions to perform local realignment of the processed sequence file to generate a re-aligned sequence file;
   (h) instructions to remove duplicate reads from the re-aligned sequence file; and
   (i) instructions to analyze coding regions of interest.
37. The method of item 36, wherein analyzing coding regions of interest comprises calling variants at every position in the regions of interest.
38. The method of item 37, wherein the regions of interest are padded by an additional 150 bases.
39. The method of item 37, wherein variant calling is performed with a modified GATK variant caller.
40. The method of item 36, wherein mapping the reads to a genomic reference sequence is performed with a Burrows Wheeler Aligner (BWA).
41. The method of item 36, wherein mapping the reads to a genomic reference sequence does not comprise soft clipping.
42. The method of item 36, wherein the genomic reference sequence is GRCh37.1 human genome reference

## Claims

1. A method for determining a presence of a variant in one or more genes in a subject comprising:
(a) obtaining a plurality of nucleic acid sequence data from a plurality of nucleic acid fragments in a nucleic acid sample;
(b) removing a portion of the plurality of nucleic acid sequence data that fails a quality threshold;
(c) mapping another portion of the plurality of nucleic acid sequence data remaining after removing the portion failing the quality threshold, to a genomic reference sequence to generate mapped data;
(d) generating a processed sequence file according to the mapped data;
(e) performing a local realignment of the processed sequence file to obtain a realigned sequence file, according to a modified genomic alignment utility limited to nucleic acid regions of interest within the one or more genes, wherein the nucleic acid regions of interest comprise coding exons +/- 50 bases of the one or more genes;
(f) removing a duplicate portion from the realigned sequence file;
(g) analyzing coding regions of interest of a portion of the realigned sequence file remaining after removing the duplicate portion; and
(h) determining the presence of the variant in the one or more genes according to the analysis of the coding regions of interest.

2. The method of claim 1, wherein the coding regions of interest for analysis comprise coding exons +/- 200 bases of the one or more genes.

3. The method of claim 1, wherein the local realignment of the processed sequence file does not include base quality score recalibration (BQSR).

4. The method of claim 1, wherein an adapter and/or molecular identification (MID) sequence is removed from the plurality of nucleic acid sequence data.

5. The method of claim 1, wherein generating the processed sequence file comprises:
(a) sorting and indexing the mapped data; and
(b) adding groups of the mapped data to a data file to generate the processed sequence file.

6. The method of claim 1, wherein analyzing the coding regions of interest comprises calling variants at every position in the coding regions of interest.

7. The method of claim 6, wherein calling the variants is performed with a modified Genome Analysis Toolkit (GATK) variant caller.

8. The method of claim 1,
wherein the nucleic acid sample comprises a plurality of nucleic acids of one or more subjects,
wherein the nucleic acid sample further comprises genomic DNA, or cDNA reversed transcribed from mRNA, and
wherein the nucleic acid sample is prepared for sequencing by performing one or more of:
(a) shearing a nucleic acid of the nucleic acid sample;
(b) concentrating the nucleic acid of the nucleic acid sample;
(c) size selecting the nucleic acid of the nucleic acid sample;
(d) repairing ends of the nucleic acid of the nucleic acid sample using a DNA polymerase;
(e) attaching one or more adapter sequences to the nucleic acid of the nucleic acid sample;
(f) amplifying the nucleic acid having an attached adapter sequence to increase its abundance in the nucleic acid sample relative to nucleic acids lacking attached adapter sequences in the nucleic acid sample; and
(g) enriching the nucleic acid sample for the nucleic acids of one or more genes of interest.

9. The method of claim 8, wherein the one or more adapter sequences comprise nucleic acid sequences for priming a sequencing reaction and/or a nucleic acid amplification reaction, wherein the one or more adapter sequences further comprise a molecular identification (MID) tag.

10. The method of claim 8, wherein enriching the nucleic acid sample for the nucleic acids of one of more genes of interests comprises capturing a nucleic acid with a nucleic acid probe or bait.

11. The method of claim 8, wherein enriching the nucleic acid sample for the nucleic acids of one or more genes of interest comprises exon capture using one or more RNA probes or baits.

12. The method of claim 11, wherein the one or more RNA probes or baits is biotinylated and specific for exonic regions, splice junction sites, or intronic regions of the one or more genes of interest, or
wherein the one or more RNA probes or baits is biotinylated and specific for a *BRCA1* gene and/or a *BRCA2* gene.

13. The method of claim 1, wherein the one or more genes comprise a *BRCA1* gene and/or a *BRCA2* gene.

14. A system comprising:
one or more electronic processors configured to:
(a) obtain a plurality of nucleic acid sequence data from a plurality of nucleic acid fragments in a nucleic acid sample;
(b) remove a portion of the plurality of nucleic acid sequence data that fails a quality threshold;
(c) map another portion of the plurality of nucleic acid sequence data remaining after removing the portion failing the quality threshold, to a genomic reference sequence to generate mapped data;
(d) generate a processed sequence file according to the mapped data;
(e) perform a local realignment of the processed sequence file to obtain a realigned sequence file, according to a modified genomic alignment utility limited to nucleic acid regions of interest within the one or more genes, wherein the nucleic acid regions of interest comprise coding exons +/- 50 bases of the one or more genes;
(f) remove a duplicate portion from the realigned sequence file; and
(g) analyze coding regions of interest of a portion of the realigned sequence file remaining after removing the duplicate portion.

15. A computer-readable medium which operates cooperatively with at least one processor to:
(a) obtain a plurality of nucleic acid sequence data from a plurality of nucleic acid fragments in a nucleic acid sample;
(b) remove a portion of the plurality of nucleic acid sequence data that fails a quality threshold;
(c) map another portion of the plurality of nucleic acid sequence data remaining after removing the portion failing the quality threshold, to a genomic reference sequence to generate mapped data;
(d) generate a processed sequence file according to the mapped data;
(e) perform a local realignment of the processed sequence file to obtain a realigned sequence file, according to a modified genomic alignment utility limited to nucleic acid regions of interest within the one or more genes, wherein the nucleic acid regions of interest comprise coding exons +/- 50 bases of the one or more genes;
(f) remove a duplicate portion from the realigned sequence file; and
(g) analyze coding regions of interest of a portion of the realigned sequence file remaining after removing the duplicate portion.
